# EUROPEAN PATENT APPLICATION

(11) **EP 4 316 243 A1**
(43) Date of publication of application: **07.02.2024**
(21) Application number: 22188936.3
(22) Date of filing: 05.08.2022
(51) Int. Cl.: A01N 43/16, A01P 1/00, A01P 3/00, A23L 3/3463, A23L 3/3481, A23L 3/3544

(54) **A PRESERVATION METHOD**

(71) Applicant: BRAIN Biotech AG, 64673 Zwingenberg (DE); Analyticon Discovery GmbH, 14473 Potsdam (DE)
(72) Inventor: KATZER, Werner, 12207 Berlin (DE); SIEMS, Karsten, 14552 Michendorf (DE); THELITZ, Axel, 13507 Berlin (DE); TIFFERT, Yvonne, 68309 Mannheim (DE)
(74) Representative: Fabry, Bernd

(57) **Abstract**

Suggested is a method for improving antimicrobial stability of a composition comprising or consisting of glycolipids, said glycolipids being selected from at least one ustilagic acid according to formula (I)-(III) or compounds 1-6.

## Description

### AREA OF INVENTION

The present invention refers to the area of glycolipids and concerns new and effective methods for preservation of preparations, by using anti-microbial agents of natural origin.

### BACKGROUND OF THE INVENTION

The rise of resistant microbes illustrates the need for further compounds able to prevent microbial infections and contaminations, while showing low toxicity in humans. Some glycolipids such as ustilagic acid have demonstrated anti-microbial properties. For instance, glycolipids have been shown to interact with the lipid bilayer of microorganisms thereby leading to deformation and collapse of the membrane skeleton. Glycolipid treatment is also associated with increased generation of reactive oxygen species (ROS) in bacteria and fungi. Accumulation of ROS occurs due to insufficient cellular detoxification. It is commonly observed in microorganisms following treatment with membrane disruptors and antibiotics and can lead to cellular apoptosis. Therefore, glycolipids show great potential for anti-microbial applications due to their high biological activity against microbes with low observed toxicity.

Glycolipids are particularly useful as food preservatives but also for other products of daily life, e.g. pet food and cosmetics. Preservation methods such as pasteurization or addition of chemical preservatives such as sorbic or benzoic acid can have a profound impact on the sensory properties of the product. However, sensory changes of products upon preservation are undesirable as customers value natural flavor. Additionally, there is a need for alternatives to synthetically produced conservatives due to the high environmental cost of precursor compounds used for industrial production. Glycolipids represent a suitable alternative to widely used preservation approaches due to their anti-microbial properties, low toxicity and more eco-friendly production.

### RELEVANT PRIOR ART

Glycolipids including cellobiose glycolipids (e.g. ustilagic acids) are well known as surfactants and showed moderate antimicrobial activity due to the disruption of the cell wall of the microorganisms, especially of yeasts and gram positive bacteria [Qin Shu et al. Pharmaceutics 2021, 13, 227, B. Mimee et al. Journal of Applied Microbiology 107 (2009) 989-996].

WO 2013 037818 A1 (WACKER) discloses ustilagic acid and its esters for fighting specific germs such as *Alicyclobacillus acidoterrestris, Penicillium expansum, Gluconacetobacter liquefaciens, Lactiplantibacillus plantarum, Zygosaccharomyces rouxii, Aspergillus brasiliensis, Brettanomyces naardanensis,* and *Weissella confusa* in non-alcoholic beverages.

### OBJECT OF THE INVENTION

Due to increasing resistance of specific microbes, particularly of germs like
- *Alicyclobacillus acidoterrestris*
- *Asaia siamensis*
- *Aspergillus brasiliensis*
- *Bacillus licheniformis*
- *Bacillus subtilis*
- *Brettanomyces sp*
- *Byssochlamys fulva*
- *Byssochlamys nivea*
- *Candida albicans*
- *Candida parapsilosis*
- *Clostridium perfringens*
- *Clostridium sporogenes*
- *Corynebacterium xerosis*
- *Dekkera naardenensis*
- *Enterococcus faecalis*
- *Gluconacetobacter liquefaciens*
- *Lactobacillus paracasei ssp. paracasei*
- *Lactobacillus plantarum ssp. plantarum*
- *Leuconostoc lactis*
- *Leuconostoc mesenteroides*
- *Malassezia furfur*
- *Neosartorya fischeri*
- *Penicillium expansum*
- *Penicillium paneum*
- *Saccharomyces cerevisiae*
- *Yarrowia lipolytica*
- *Zygosaccharomyces bailii*
- *Zygosaccharomyces rouxii*

which are found in many products of daily life, there is demand for new and highly effective preservatives, particularly obtained from natural sources and capable of fighting these microbes, preferably at a very low application level.

The preservative composition of the present invention is preferably applied for food products excluding beverages.

### BRIEF DESCRIPTION OF THE INVENTION

A first object of the present invention refers to a method for improving antimicrobial stability of a composition, such as cosmetic, detergent or food preparations, against at least one of the following micro-organisms
- *Alicyclobacillus acidoterrestris*
- *Asaia siamensis*
- *Aspergillus brasiliensis*
- *Bacillus licheniformis*
- *Bacillus subtilis*
- *Brettanomyces sp*
- *Byssochlamys fulva*
- *Byssochlamys nivea*
- *Candida albicans*
- *Candida parapsilosis*
- *Clostridium perfringens*
- *Clostridium sporogenes*
- *Corynebacterium xerosis*
- *Dekkera naardenensis*
- *Enterococcus faecalis*
- *Gluconacetobacter liquefaciens*
- *Lactobacillus paracasei ssp. paracasei*
- *Lactobacillus plantarum ssp. plantarum*
- *Leuconostoc lactis*
- *Leuconostoc mesenteroides*
- *Malassezia furfur*
- *Neosartorya fischeri*
- *Penicillium expansum*
- *Penicillium paneum*
- *Saccharomyces cerevisiae*
- *Yarrowia lipolytica*
- *Zygosaccharomyces bailii*
- *Zygosaccharomyces rouxii*
comprising or consisting of the following steps:
(a) providing a composition in need of such improved antimicrobial stability;
(b) providing at least one glycolipid and optionally at least one other antimicrobial agent;
(c) adding a working amount of said at least one glycolipid and optionally said at least one other anti-microbial agent to said composition;
(d) optionally providing at least one other compound to said composition affecting solubility of compounds added in step (b) and (c);
(e) adjusting the pH of the composition thus obtained to about 1 to about 4,
wherein said glycolipid represents an ustilagic acid or an ester of ustilagic acid esterified with a linear C₁-C₄ aliphatic alcohol.

### Glycolipids

Glycolipids are molecules with at least one glycosidic bond between mono- or oligosaccharides and lipid molecules. Several classes of glycolipids have been identified such as rhamnolipids, trehaloselipids, cellobioselipids and mannosylerythritolipids.

Ustilagic acid is an organic compound with the formula C₃₆H₆₄O₁₈. The acid is a cellobiose lipid produced by the corn smut fungus *Ustilago maydis* under conditions of nitrogen starvation. The acid was discovered in 1950 and was proven to be an amphipathic glycolipid with surface active properties. The name comes from Latin *ustus* which means *burnt* and refers to the scorched appearance of the smut fungi. Yeast species of the genus *Pseudozyma* are known to produce ustilagic acid. For example, the yeasts *Pseudozyma fusiformata* and *Pseudozyma graminicola* secrete ustilagic acids, 2-O-3-hydroxyhexanoyl-beta-D-glucopyranosyl-(1→4)-6-O-acetyl-beta-D-glucopyranosyl-(1→16)-2,15,16-trihydroxyhexadecanoic acid. Similar compounds are the extracellular cellobiose lipids of the yeasts *Cryptococcus humicola* and *Tricho-sporon porosum* : 2,3,4-O-triacetyl-beta-D-glucopyranosyl-(1→4)-6-O-acetyl-beta-D-glucopyranosyl -(1→16)-2,16-dihydroxyhexa decanoic acid.

Some glycolipids can be used as natural conservatives without changing the sensatory properties of the product. Ustilagic acids have been shown to possess antibiotic properties (US2698843). The biosynthesis of ustilagic acid as well as its use as compound controlling the phytopathogen *Botrytis cinerea* have been reported before (http ://archiv.ub.uni-marburg .de/opus/frontdoor .php?source gpus=234 2&la=de).

Surprisingly it has been found that the glycolipids according to the present invention are active against a broad spectrum of microorganisms, including bacteria, fungi and yeasts, particularly
- *Alicyclobacillus acidoterrestris*
- *Asaia siamensis*
- *Aspergillus brasiliensis*
- *Bacillus licheniformis*
- *Bacillus subtilis*
- *Brettanomyces sp*
- *Byssochlamys fulva*
- *Byssochlamys nivea*
- *Candida albicans*
- *Candida parapsilosis*
- *Clostridium perfringens*
- *Clostridium sporogenes*
- *Corynebacterium xerosis*
- *Dekkera naardenensis*
- *Enterococcus faecalis*
- *Gluconacetobacter liquefaciens*
- *Lactobacillus paracasei ssp. paracasei*
- *Lactobacillus plantarum ssp. plantarum*
- *Leuconostoc lactis*
- *Leuconostoc mesenteroides*
- *Malassezia furfur*
- *Neosartorya fischeri*
- *Penicillium expansum*
- *Penicillium paneum*
- *Saccharomyces cerevisiae*
- *Yarrowia lipolytica*
- *Zygosaccharomyces bailii*
- *Zygosaccharomyces rouxii*
already at very low working levels given that the respective compositions are adjusted to low pH values of from about 1 to about 4, preferably of from about 1 to about 3 and more preferably of about 2 to about 2.5 The products are typically obtained or obtainable by extraction of natural sources.

Glycolipids according to the present invention are known to possess excellent emulsifying and surfactant properties. Therefore, in a preferred embodiment, the glycolipids according to the present invention substitute or support emulsifiers and surfactant present in compositions according to the present invention.

### Preferred glycolipids

In a preferred embodiment the glycolipids according to the present invention follow formula (I) 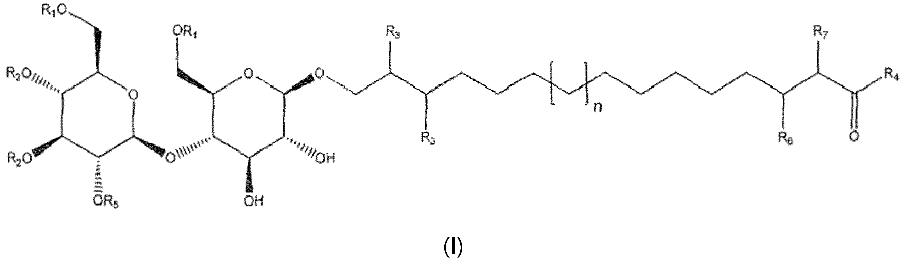 wherein independently from each other
- n: stands for 1 or 2,
- R₁: represents hydrogen or -COCH₃;
- R₂: represents H or 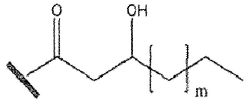
m = 1, 2 or 3;
- R₃: represents hydrogen or -OH;
- R₄: represents -OH or-OCH3;
- R₅: represents hydrogen or 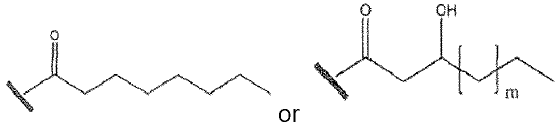
m = 1,2 or 3;
- R₆: represents hydrogen or -OH;
- R₇: represents hydrogen or -OH or =O;
or a salt of said glycolipid.

In a preferred embodiment, the glycolipids according to the present invention follow formula (II) 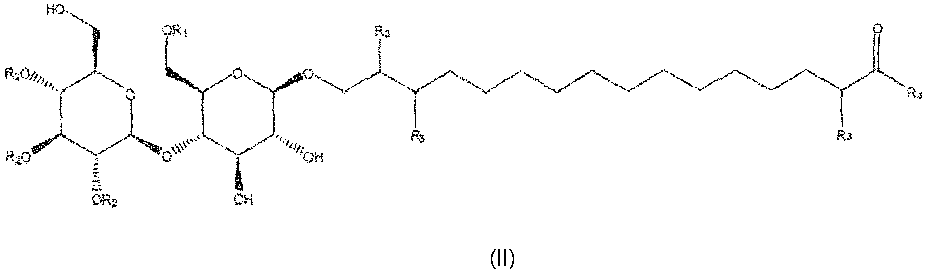 wherein independently from each other
- R₁: represents hydrogen or -COCH₃;
- R₂: represents H or 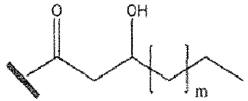
m = 1, 2 or 3;
- R₃: represents hydrogen or -OH;
- R₄: represents -OH or-OCH3;
or a salt of said glycolipid.

In a preferred embodiment, the glycolipids according to the present invention follow formula (III) 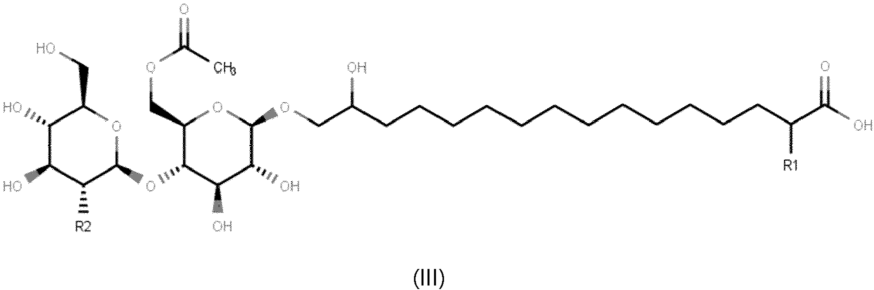 wherein independently from each other
- R₁: represents hydrogen or -OH;
- R₂: represents hydrogen or 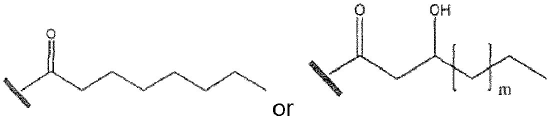
m = 1,2 or 3; or a salt of said glycolipid.

Surprisingly, while all antimicrobial compounds of the present invention are active against most of the microorganisms mentioned above, certain compounds show an exceptionally high and broad activity. In particular compounds following the general structure of formula III are able to inhibit growth and viability of microorganisms at even lower working concentrations compared to other glycolipids.

Compounds 1 to 6, following the general formula of formula (III), in particular exhibit excellent antimicrobial properties at low concentrations. In a preferred embodiment the glycolipids according to the present invention are selected from the following formulae of Compound 1 to 6:

| **Compound** | **Structure** | **Formula** | **Molecular weight** | **CAS** |
|---|---|---|---|---|
| 1 | 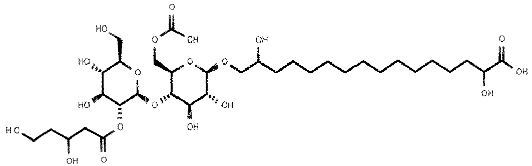 | C₃₆H₆₄O₁₈ | 784,89 | 867578-35-6 |
| 2 | 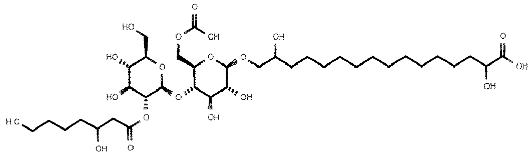 | C₃₈H₆₈O₁₈ | 812,94 | 1427087-16-8 |
| 3 | 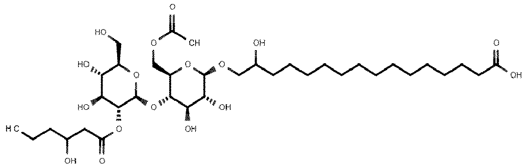 | C₃₆H₆₄O₁₇ | 768,89 | 1427028-86-1 |
| 4 | 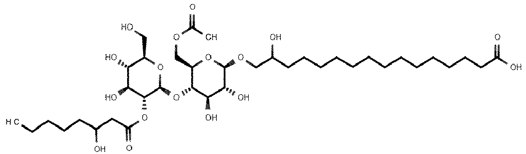 | C₃₈H₆₈O₁₇ | 796,95 | |
| 5 | 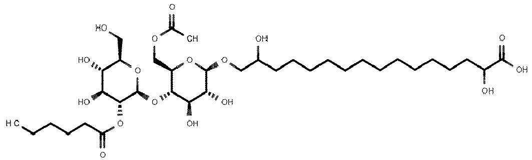 | C₃₆H₆₄O₁₇ | 768,89 | |
| 6 | 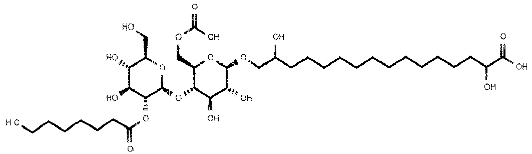 | C₃₈H₆₈O₁₇ | 796,95 | 1427039-33-5 |

or a salt of said glycolipid.

In a preferred embodiment the salts according to the present invention represent alkali salts, alkaline salts, alkaline earth salts, ammonium salts, alkanol ammonium salts, glucammonium salts or mixtures thereof.

In a preferred embodiment at least one glycolipid according to the present invention is added in an amount of from 1 to 1,000 ppm - calculated on composition.

In a preferred embodiment at least one glycolipid according to the present invention is added in an amount of from 10 to 200 ppm - calculated on composition.

In a preferred embodiment of the method the pH value of the composition is adjusted by inorganic or organic salts.

### Secondary additional anti-microbial compounds

In a preferred embodiment the preservative method according to the present invention may include blends of said glycolipids, additional antimicrobial compounds different from said glycolipids as they are for example listed in REGULATION (EC) No 1333/2008 OF THE EUROPEAN PARLIAMENT AND OF THE COUNCIL of 16 December 2008 on food additives or in REGULATION (EC) No 1223/2009 OF THE EUROPEAN PARLIAMENT AND OF THE COUNCIL of 30 November 2009 on cosmetic products as well as further compounds affecting solubility of said glycolipids such as saponins or emulsifiers listed in EU 1333/2008 or EU 1223/2009.

Suitable species can be selected from the group consisting of preservatives selected from the group consisting of benzoic acid and para-hydroxybenzoic acid, their esters and salts, benzyl benzoate, propionic acid and its salts, salicylic acid and its salts, 2,4-hexadienoic acid (sorbic acid) and its salts, levulinic acid and its salts, anisic acid and its salts, perillic acid and its salts, cinnamic acid and its salts, formaldehyde and paraformaldehyde, 4-hydroxy benzaldehyde, ortho-, meta-, and para-anisic aldehyde, cinnamic aldehyde, cinnamic alcohol, 2-hydroxybiphenyl ether and its salts, 2-zinc-sulfidopyridine N-oxide, inorganic sulfites and bisulfites, sodium iodate, chlorobutanolum, 4-ethylmercury-(II)5-amino-1,3-bis(2-hydroxybenzoic acid), its salts and esters, dehydracetic acid, formic acid, 1,6-bis(4-amidino-2-bromophenoxy)-n-hexane and its salts, the sodium salt of ethylmercury-(II)-thiosalicylic acid, phenylmercury and its salts, 10-undecylenic acid and its salts, 5-amino-1,3-bis(2-ethylhexyl)-5-methyl-hexa-hydropyrimidine, 5-bromo-5-nitro-1,3-dioxane, 2-bromo-2-nitro-1,3-propanediol, 2,4-dichlorobenzyl alcohol, N-(4-chlorophenyl)-N'-(3,4-dichlorophenyl)-urea, 4-chloro-m-cresol, 2,4,4'-trichloro-2'-hydroxy-diphenyl ether, 4-chloro-3,5-dimethylphenol, 1,1'-methylene-bis(3-(1-hydroxymethyl-2,4-dioximidazolidin-5-yl)urea), poly-(hexamethylenediguanide) hydrochloride, (Benzyloxymethoxy)-methanol hexamethylenetetramine, 1-(3-chloroallyl)-3,5,7-triaza-1-azonia-adamantane chloride, 1-(4-chlorophenoxy)-1-(1H-imidazol-1-yl)-3,3-dimethyl-2-butanone, 1,3-bis-(hydroxymethyl)-5,5-dimethyl-2,4-imidazolidinedione, 1,2-dibromo-2,4-dicyanobutane, 2,2'-methylene-bis(6-bromo-4-chlorophenol), bromochlorophene, mixture of 5-chloro-2-methyl-3(2H)-isothiazolinone, 2-methyl-3(2H)-isothiazolinone and with magnesium chloride and magnesium nitrate, 2-Octyl-2H-isothiazol-3-one, 1,2-benzisothiazol-3(2H)-one, 2-benzyl-4-chlorophenol, 3-(4-Chlorphenoxy)-1,2-propanediol (Chlorphenesin), 2-chloroacetamide, chlorhexidine, chlorhexidine acetate, chlorhexidine gluconate, chlorhexidine hydrochloride, N-alkyl(C₁₂-C₂₂)trimethyl-ammonium bromide and chloride, 4,4-dimethyl-1,3-oxazolidine, N-hydroxymethyl-N-(1,3-di(hydroxymethyl)-2,5-dioxoimidazolidin-4-yl)-N'-hydroxymethylurea, 1,6-bis(4-amidino-phenoxy)-n-hexane and its salts, glutaraldehyde, 5-ethyl-1-aza-3,7-dioxabicyclo(3.3.0)octane, 3-(4-chlorophenoxy)-1,2-propanediol, hyamines, alkyl-(C₈-C₁₈)-dimethyl-benzyl-ammonium chloride, alkyl-(C₈-C₁₈)-dimethyl-benzylammonium bromide, alkyl-(C₈-C₁₈)-dimethyl-benzyl-ammonium saccharinate, benzyl hemiformal, 3-iodo-2-propynyl butylcarbamate, sodium hydroxymethyl-aminoacetate or sodium hydroxymethyl-aminoacetate, imidazolidinylurea, diazolidinylurea, sodium hydroxymethylglycinate, DMDM hydantoin, Tropolone, (Ethylendioxy)dimethanol, 2-Brom-2-(brommethyl)pentandinitril, N-(3-Aminopropyl)-N-dodecylpropan-1,3-diamin, α,α',α"-trimethyl-1,3,5-triazine-1,3,5(2H,4H,6H)-triethanol, pyridine-2-thiol-1-oxide, sodium salt, Tetrahydro-1,3,4,6-tetrakis(hydroxymethyl)imidazo[4,5-d]11erillart-2,5(1H,3H)-dion, 1,3-bis(hydroxymethyl) -1-(1,3,4-tris(hydroxy-methyl)-2,5-dioxoimidazolidin-4-yl)urea (Diazolidinyl Urea), 1,3-Bis(hydroxy-methyl)-5,5-dimethylimidazolidine-2,4-dione, 3-Acetyl-2-hydroxy-6-methyl-4*H*-pyran-4-one, cetyl pyridium chloride, caprylhydroxamic acid, sorbohydroxamic acid and their mixtures; 1,3-propanediol, methyl propanediol, 1,2-pentanediol, 1,2-hexanediol, 1,2-octanediol, 1,2-decanediol, 1,5-pentanediol, 1,6-hexanediol, 1,8-octanediol, 1,2-decanediol, ethylhexylglycerin, hexoxy-propan-1,2-diol, heptoxy- propan-1,2-diol, octoxy-propan-1,2-diol, 3-phenoxy-propan-1,2-diol, 3-benzyloxypropan-1,2-diol, 3-phenylethyloxy-propan-1,2-diol, 3-phenylpropyloxy-propan-1,2-diol, 3-methylbenzyloxy-propan-1,2-diol, sorbitan caprylate, triclosan, climbazole, Octopirox (1-hydroxy-4-methyl-6-(2,4,4-trimethylpentyl)-2(1H)-pyridone, 2-aminoethanol), chitosan, farnesol, 2-butyloctanoic acid, 2-Benzylheptan-1-ol, glycerol monolaurate, bis(2-pyridylthio)zinc 1,1'-dioxide, N,N'-(decane-1,10-diyldipyridin-1-yl-4-ylidene)-dioctan-1-amine dihydrochloride (octenidine dihydrochloride), thymol, eugenol, benzyl alcohol, 2-phenyethyl alcohol, 3-phenyl propanol, 2-phenoxyethanol, 1-phenoxy-propan-2-ol, 3-phenoxypropanol, benzyloxymethanol, 4-hydroxyacetophenone, lactic acid, ethanol, PHB (esters). and mixtures thereof.

In a preferred embodiment, secondary additional anti-microbial compounds are selected from the group of benzoic acid and 2,4-hexadienoic acid (sorbic acid), lactic acid, ethanol and para-hydroxybenzoic acid (PHB), their esters and salts.

### Solubilizers

A further objective of the present invention has been improving solubility of glycolipids and their mixtures in aqueous solutions depending on the selected glycolipids and other compounds as well as the desired pH value. It is therefore beneficial for the present invention to further add compounds that increase solubility for different combinations of glycolipids at various pH values. For instance, ustilagic acid solubility is decreased at low pH values. Surprisingly, addition of plant-derived saponins greatly increases solubility at lower pH values and provide additional anti-microbial properties.

In a preferred embodiment the preservative method according to the present invention may include compounds increasing solubility of glycolipids selected from the group consisting of saponines obtainable from the plant genera
- *Quiallaja,*
- *Yucca,*
- *Dioscorea,*
- *Paris,*
- *Hosta,*
- *Agave,*
- *Asparagus,*
- *Allium,*
- *Sapindus,*
- *Aesculus,*
- *Hedera,*
- *Blighia,*
- *Dipsacus,*
- *Aralia* and/or
- *Anemone*
or mixtures thereof, emulgators E400 to E499 approved for food preparations in the EU, emulgators, synthetic food grade emulsifiers selected from the group consisting of lecithin; polyglycerol esters (PGE), polysorbates, stearoyl lactylates, propylene glycol esters (PGMS), sucrose esters; polyglycerol Polyricinoleate (PGPR); ammonium phosphatide (AMP); mono and diglycerides of C6-C22 saturated or unsaturated fatty acids and mixtures thereof.

### FOOD PREPARATIONS

Food compositions according to the invention are any preparations or compositions comprising the compositions according to the invention which are suitable for consumption and are used for nutrition or enjoyment purposes, and are generally products which are intended to be introduced into the human or animal oral cavity, to remain there for a certain time and then either be eaten (e.g. ready-to-eat foodstuffs or feeds, see also herein below) or removed from the oral cavity again (e.g. chewing gums). Such products include any substances or products which in the processed, partially processed or unprocessed state are to be ingested by humans or animals. They also include substances which are added to orally consumable products during their manufacture, preparation or treatment and which are intended to be introduced into the human or animal oral cavity.

The food compositions according to the invention also include substances which in the unchanged, treated or prepared state are to be swallowed by a human or animal and then digested; in this respect, the orally consumable products according to the invention also include casings, coatings or other encapsulations which are to be swallowed at the same time or which may be expected to be swallowed. The expression "orally consumable product" covers ready-to-eat foodstuffs and feeds, that is to say foodstuffs or feeds that are already complete in terms of the substances that are important for the taste. The expressions "ready-to-eat foodstuff" and "ready-to-eat feed" also include solid or semi-solid ready-to-eat foodstuffs or feeds. Examples which may be mentioned are frozen products, which must be thawed and heated to eating temperature before they are eaten. Products such as yoghurt or ice-cream as well as chewing gums or hard caramels are also included among the ready-to-eat foodstuffs or feeds.

Preferred food compositions according to the invention also include "semi-finished products". Within the context of the present text, a semi-finished product is to be understood as being an orally consumable product which, because of a very high content of flavorings and taste-imparting substances, is unsuitable for use as a ready-to-eat orally consumable product (in particular foodstuff or feed). Only by mixing with at least one further constituent (e.g. by reducing the concentration of the flavorings and taste-imparting substances in question) and optionally further process steps (e.g. heating, freezing) is the semi-finished product converted into a ready-to-eat orally consumable product (in particular foodstuff or feed). Examples of semi-finished products which may be mentioned here are

Food composition according to the invention preferably comprises one or more preparations for nutrition or enjoyment purposes. These include in particular (reduced-calorie) baked goods (e.g. bread, dry biscuits, cakes, other baked articles), confectionery (e.g. chocolates, chocolate bars, other products in bar form, fruit gums, dragées, hard and soft caramels, chewing gum), meat products (e.g. ham, fresh sausage or raw sausage preparations, spiced or marinated fresh or salt meat products), eggs or egg products (dried egg, egg white, egg yolk), cereal products (e.g. breakfast cereals, muesli bars, precooked ready-to-eat rice products), dairy products (e.g. rice pudding, yoghurt, kefir, cream cheese, soft cheese, hard cheese, dried milk powder, whey, butter, buttermilk, partially or completely hydrolysed milk-protein-containing products), products made from soy protein or other soybean fractions (e.g. soy milk and products produced therefrom, preparations containing soy lecithin, fermented products such as tofu or tempeh or products produced therefrom and mixtures with fruit preparations and optionally flavors), fruit preparations (e.g. jams, sorbets, fruit sauces, fruit fillings), vegetable preparations (e.g. ketchup, sauces, dried vegetables, frozen vegetables, precooked vegetables, boiled-down vegetables), snacks (e.g. baked or fried potato crisps or potato dough products, maize- or groundnut-based extrudates), fat- and oil-based products or emulsions thereof (e.g. mayonnaise, remoulade, dressings, in each case full-fat or reduced-fat), other ready-made dishes and soups (e.g. dried soups, instant soups, precooked soups), spices, spice mixtures and in particular seasonings which are used, for example, in the snacks field, sweetener preparations, tablets or sachets, other preparations for sweetening or whitening foods. The preparations within the scope of the invention can also be used in the form of semi-finished products for the production of further preparations for nutrition or enjoyment purposes. The preparations within the scope of the invention can also be in the form of capsules, tablets (uncoated and coated tablets, e.g. enteric coatings), dragées, granules, pellets, solids mixtures, dispersions in liquid phases, in the form of emulsions, in the form of powders, in the form of solutions, in the form of pastes, or in the form of other preparations which can be swallowed or chewed, and in the form of food supplements.

The preparations can also be in the form of capsules, tablets (uncoated and coated tablets, e.g., enteric coatings), dragées, granules, pellets, solids mixtures, dispersions in liquid phases, in the form of emulsions, in the form of powders, in the form of solutions, in the form of pastes, or in the form of other preparations which can be swallowed or chewed, for example in the form of food supplements.

The semi-finished products are generally used for the production of ready-to-use or ready-to-eat preparations for nutrition or enjoyment purposes.

Further constituents of a ready-to-eat preparation or semi-finished product for nutrition or enjoyment purposes can be conventional base substances, auxiliary substances and additives for foods or enjoyment foods, for example water, mixtures of fresh or processed, vegetable or animal base or raw substances (e.g. raw, roast, dried, fermented, smoked and/or boiled meat, bone, cartilage, fish, vegetables, herbs, nuts, vegetable pastes or mixtures thereof), digestible or non-digestible carbohydrates (e.g. sucrose, maltose, fructose, glucose, dextrins, amylose, amylopectin, inulin, xylans, cellulose, tagatose), sugar alcohols (e.g. sorbitol, erythritol), natural or hardened fats (e.g. tallow, lard, palm fat, cocoa fat, hardened vegetable fat), oils (e.g. sunflower oil, groundnut oil, maize germ oil, olive oil, fish oil, soya oil, sesame oil), fatty acids or their salts (e.g. potassium stearate), proteinogenic or non-proteinogenic amino acids and related compounds (e.g. γ-aminobutyric acid, taurine), peptides (e.g. glutathione), natural or processed proteins (e.g. gelatin), enzymes (e.g. peptidases), nucleic acids, nucleotides, taste correctors for unpleasant taste impressions, further taste modulators for further, generally not unpleasant taste impressions, other taste-modulating substances (e.g. inositol phosphate, nucleotides such as guanosine monophosphate, adenosine monophosphate or other substances such as sodium glutamate or 2-phenoxypropionic acid), emulsifiers (e.g. lecithins, diacylglycerols, gum arabic), stabilisers (e.g. carrageenan, alginate), preservatives (e.g. benzoic acid and its salts, sorbic acid and its salts), antioxidants (e.g. tocopherol, ascorbic acid), chelators (e.g. citric acid), organic or inorganic acidifying agents (e.g. acetic acid, phosphoric acid), additional bitter substances (e.g. quinine, caffeine, limonene, amarogentine, humulone, lupulone, catechols, tannins), substances that prevent enzymatic browning (e.g. sulfite, ascorbic acid), ethereal oils, plant extracts, natural or synthetic colourings or colouring pigments (e.g. carotinoids, flavonoids, anthocyans, chlorophyll and derivatives thereof), spices, trigeminally active substances or plant extracts containing such trigeminally active substances, synthetic, natural or nature-identical flavourings or odorants as well as odour correctors.

Food compositions according to the invention, for example those in the form of preparations or semi-finished products, preferably comprise a flavour composition in order to complete and refine the taste and/or odour. A preparation can comprise as constituents a solid carrier and a flavour composition. Suitable flavour compositions comprise, for example, synthetic, natural or nature-identical flavourings, odorants and taste-imparting substances, reaction flavourings, smoke flavourings or other flavour-giving preparations (e.g. protein (partial) hydrolysates, preferably protein (partial) hydrolysates having a high arginine content, barbecue flavourings, plant extracts, spices, spice preparations, vegetables and/or vegetable preparations) as well as suitable auxiliary substances and carriers. Particularly suitable here are the flavour compositions or constituents thereof which produce a roasted, meaty (in particular chicken, fish, seafood, beef, pork, lamb, mutton, goat), vegetable-like (in particular tomato, onion, garlic, celery, leek, mushroom, aubergine, seaweed), spicy (in particular black and white pepper, cardamom, nutmeg, pimento, mustard and mustard products), fried, yeast-like, boiled, fatty, salty and/or pungent flavour impression and accordingly can enhance the spicy impression. The flavour compositions generally comprise more than one of the mentioned ingredients.

The food compositions of the present invention are preferably selected from the group comprising
- confectionery, preferably reduced-calorie or calorie-free confectionery, preferably selected from the group comprising muesli bar products, fruit gums, dragées, hard caramels and chewing gum,
- cereal products, preferably selected from the group comprising low-sugar and sugar-free breakfast cereals and muesli bars,
- dairy products, preferably selected from the group comprising reduced-fat and fat-free yoghurt, kefir, whey, buttermilk and ice-cream,
- products made from soy protein or other soybean fractions, preferably selected from the group comprising soy milk, products produced from soy milk, preparations containing soy lecithin, products produced from preparations containing soy lecithin and mixtures with fruit preparations and optionally flavours,
- sweetener preparations, tablets and sachets,
- sugar-free dragées,
- ice-cream, with or without milk-based constituents, preferably sugar-free.

### Food grade emulsifiers

The preferred food grade emulsifiers encompass the following groups:
- Lecithin;
- Fatty acid derivatives, such as for example polyglycerol esters (PGE), polysorbates ("TWEEN"), stearoyl lactylates, propylene glycol esters (PGMS), and sucrose esters;
- Polyglycerol Polyricinoleate (PGPR);
- Ammonium Phosphatide (AMP);
- Mono and Diglycerides of C₆-C₂₂ saturated or unsaturated fatty acids;
- Saponins or extracts prepared from saponin-containing plants, e.g. Quillaia extract (E999)
and mixtures thereof.

### Aroma or flavoring compounds

Aroma compounds and flavoring agents are well known in the art and can be chosen from synthetic flavoring liquid and/or oils derived from plants leaves, flowers, fruits and so forth, and combinations thereof. Representative flavoring liquids include: artificial, natural or synthetic fruit flavors such as eucalyptus, lemon, orange, banana, grape, lime, apricot and grapefruit oils and fruit essences including apple, strawberry, cherry, orange, pineapple and so forth; bean and nut derived flavors such as coffee, cocoa, cola, peanut, almond and so forth; and root derived flavors such as licorice or ginger.

Suitable aroma or flavoring compounds are listed in REGULATION (EC) No 1334/2008 OF THE EUROPEAN PARLIAMENT AND OF THE COUNCIL of 16 December 2008 on flavorings and certain food ingredients with flavoring properties for use in and on foods and amending Council Regulation (EEC) No 1601/91, Regulations (EC) No 2232/96 and (EC) No 110/2008 and Directive 2000/13/EC.

### Sweeteners

The term "sweeteners" here denotes substances having a relative sweetening power of at least 25, based on the sweetening power of sucrose (which accordingly has a sweetening power of 1). Sweeteners to be used in an orally consumable product (in particular foodstuff, feed or medicament) according to the invention (a) are preferably non-cariogenic and/or have an energy content of not more than 5 kcal per gram of the orally consumable product.

Advantageous sweeteners in a preferred orally consumable product (in particular foodstuff, feed or medicament) according to the invention are selected from the following groups:
(a) Naturally occurring sweeteners, preferably selected from the group comprising miraculin, monellin, mabinlin, thaumatin, curculin, brazzein, pentaidin, D-phenylalanine, D-tryptophan, and extracts or fractions obtained from natural sources, comprising those amino acids and/or proteins, and the physiologically acceptable salts of those amino acids and/or proteins, in particular the sodium, potassium, calcium or ammonium salts, neohesperidin dihydrochalcone, naringin dihydrochalcone, stevioside, steviolbioside, rebaudiosides, in particular rebaudioside A, rebaudioside B, rebaudioside C, rebaudioside D, rebaudioside E, rebaudioside F, rebaudioside G, rebaudioside H, dulcosides and rubusoside, suavioside A, suavioside B, suavioside G, suavioside H, suavioside I, suavioside J, baiyunoside 1, baiyunoside 2, phlomisoside 1, phlomisoside 2, phlomisoside 3 and phlomisoside 4, abrusoside A, abrusoside B, abrusoside C, abrusoside D, cyclocaryoside A and cyclocaryoside I, osladin, polypodoside A, strogin 1, strogin 2, strogin 4, selligueain A, dihydroquercetin 3-acetate, perillartin, telosmoside A₁₅, periandrin I-V, pterocaryosides, cyclocaryosides, mukuroziocides, trans-anethole, trans-cinnamaldehyde, bryosides, bryonosides, bryonodulcosides, carnosiflosides, scandenosides, gypenosides, trilobatin, phloridzin, dihydroflavanols, hematoxylin, cyanin, chlorogenic acid, albiziasaponin, telosmosides, gaudichaudioside, mogrosides, mogroside V, hernandulcins, monatin, phyllodulcin, glycyrrhetinic acid and derivatives thereof, in particular glycosides thereof such as glycyrrhizine, and the physiologically acceptable salts of those compounds, in particular the sodium, potassium, calcium or ammonium salts; and extracts or concentrated fractions of the extracts, selected from the group comprising thaumatococcus extracts (katamfe plant), extracts from *Stevia* ssp. (in particular *Stevia rebaudiana*), swingle extracts (*Momordica* or *Siratia grosvenorii,* Luo-Han-Guo), extracts from *Glycerrhyzia* ssp. (in particular *Glycerrhyzia glabra*), extracts from *Rubus* ssp. (in particular *Rubus suavissimus*), citrus extracts and extracts from *Lippia dulcis;*
(b) Synthetic sweet-tasting substances, preferably selected from the group comprising magap, sodium cyclamate or other physiologically acceptable salts of cyclamic acid, acesulfame K or other physiologically acceptable salts of acesulfame, neohesperidin dihydrochalcone, naringin dihydrochalcone, saccharin, saccharin sodium salt, aspartame, superaspartame, neotame, alitame, advantame, perillartin, sucralose, lugduname, carrelame, sucrononate and sucrooctate.

### Thickeners

Advantageous thickeners in a preferred orally consumable product (in particular foodstuff, feed or medicament) according to the invention are selected from the group comprising: crosslinked polyacrylic acids and derivatives thereof, polysaccharides and derivatives thereof, such as xanthan gum, agar-agar, alginates or tyloses, cellulose derivatives, for example carboxymethylcellulose or hydroxycarboxymethylcellulose, fatty alcohols, monoglycerides and fatty acids, polyvinyl alcohol and polyvinylpyrrolidone.

Preference is given according to the invention to an orally consumable product (in particular foodstuff or feed) which comprises milk thickened with lactic acid bacteria and/or cream thickened with lactic acid bacteria and which preferably is selected from the group comprising yoghurt, kefir and quark.

A food composition according to the invention comprising milk thickened with lactic acid bacteria and/or cream thickened with lactic acid bacteria is advantageously an orally consumable product which comprises a probiotic, wherein the probiotic is preferably selected from the group comprising Bifidobacterium animalis subsp. lactis BB-12, Bifidobacterium animalis subsp. lactis DN-173 010, Bifidobacterium animalis subsp. lactis HN019, Lactobacillus acidophilus LA5, Lactobacillus acidophilus NCFM, Lactobacillus johnsonii La1, Lactobacillus casei immunitass/defensis, Lactobacillus casei Shirota (DSM 20312), Lactobacillus casei CRL431, Lactobacillus reuteri (ATCC 55730) and Lactobacillus rhamnosus (ATCC 53013).

### Additives for chewing gums

Particular preference is given to an orally consumable product (in particular foodstuff, feed or medicament) according to the invention that is a chewing gum and comprises a chewing-gum base. The chewing-gum base is preferably selected from the group comprising chewing-gum or bubble-gum bases. The latter are softer, so that gum bubbles can also be formed therewith. Preferred chewing-gum bases according to the invention include, in addition to the natural resins or the natural latex chicle that are traditionally used, elastomers such as polyvinyl acetate (PVA), polyethylene, (low or medium molecular weight) polyisobutene (PIB), polybutadiene, isobutene-isoprene copolymers (butyl rubber), polyvinyethyl ether (PVE), polyvinylbutyl ether, copolymers of vinyl esters and vinyl ethers, styrene-butadiene copolymers (styrene-butadiene rubber, SBR) or vinyl elastomers, for example based on vinyl acetate/vinyl laurate, vinyl acetate/vinyl stearate or ethylene/vinyl acetate, as well as mixtures of the mentioned elastomers, as described, for example, in EP 0 242 325, US 4,518,615, US 5,093,136, US 5,266,336, US 5,601,858 or US 6,986,709. In addition, chewing-gum bases that are preferably to be used according to the invention preferably comprise further constituents such as, for example, (mineral) fillers, plasticisers, emulsifiers, antioxidants, waxes, fats or fatty oils, such as, for example, hardened (hydrogenated) vegetable or animal fats, mono-, di- or tri-glycerides. Suitable (mineral) fillers are, for example, calcium carbonate, titanium dioxide, silicon dioxide, talcum, aluminium oxide, dicalcium phosphate, tricalcium phosphate, magnesium hydroxide and mixtures thereof. Suitable plasticisers, or agents for preventing adhesion (detackifiers), are, for example, lanolin, stearic acid, sodium stearate, ethyl acetate, diacetin (glycerol diacetate), triacetin (glycerol triacetate), triethyl citrate. Suitable waxes are, for example, paraffin waxes, candelilla wax, carnauba wax, microcrystalline waxes and polyethylene waxes. Suitable emulsifiers are, for example, phosphatides such as lecithin, mono- and di-glycerides of fatty acids, for example glycerol monostearate.

Chewing gums according to the invention (in particular as disclosed above) preferably comprise constituents such as sugars of different types, sugar substitutes, other sweet-tasting substances, sugar alcohols (in particular sorbitol, xylitol, mannitol), ingredients having a cooling effect, taste correctors for unpleasant taste impressions, further taste-modulating substances (e.g. inositol phosphate, nucleotides such as guanosine monophosphate, adenosine monophosphate or other substances such as sodium glutamate or 2-phenoxypropionic acid), humectants, thickeners, emulsifiers, stabilisers, odour correctors and flavours (e.g. eucalyptusmenthol, cherry, strawberry, grapefruit, vanilla, banana, citrus, peach, blackcurrant, tropical fruits, ginger, coffee, cinnamon, combinations (of the mentioned flavours) with mint flavours as well as spearmint and peppermint on their own). The combination *inter alia* of the flavours with further substances that have cooling, warming and/or mouth-watering properties is of particular interest.

### COSMETIC PREPARATIONS

Another object of the present invention refers to a skin care, personal care, sun care or hair care product or product formulation, comprising the composition as defined above. The preparations may represent for example a cosmetic cream, lotion, spray, emulsion, ointment, gel or mouse and the like.

The preparations according to the invention may contain antidandruff agents, irritation-preventing agents, irritation-inhibiting agents, antioxidants, adstringents, perspiration-inhibiting agents, antiseptic agents, ant-statics, binders, buffers, carrier materials, chelating agents, cell stimulants, cleansing agents, care agents, deodorizing agents, antiperspirants, softeners, emulsifiers, enzymes, essential oils, fibres, film-forming agents, fixatives, foam-forming agents, foam stabilizers, substances for preventing foaming, foam boosters, gelling agents, gel-forming agents, hair care agents, hair-setting agents, hair-straightening agents, moisture-donating agents, moisturizing substances, moisture-retaining substances, bleaching agents, strengthening agents, stain-removing agents, optically brightening agents, impregnating agents, dirt-repellent agents, friction-reducing agents, lubricants, moisturizing creams, ointments, opacifying agents, plasticizing agents, covering agents, polish, gloss agents, polymers, powders, proteins, re-oiling agents, abrading agents, silicones, hair promotion agents, cooling agents, skin-cooling agents, warming agents, skin-warming agents, stabilizers, UV-absorbing agents, UV filters, detergents, thickeners, vitamins, oils, waxes, fats, phospholipids, saturated fatty acids, mono- or polyunsaturated fatty acids, α-hydroxy acids, polyhydroxyfatty acids, liquefiers, dyestuffs, color-protecting agents, pigments, odoriferous substances, polyols, surfactants, electrolytes, organic solvents or silicone derivatives and the like as additional auxiliaries and additives.

### INDUSTRIAL APPLICATION

The present invention also encompasses the use of glycolipids as antimicrobial agents against at least one of the following micro-organisms
- *Alicyclobacillus acidoterrestris*
- *Asaia siamensis*
- *Aspergillus brasiliensis*
- *Bacillus licheniformis*
- *Bacillus subtilis*
- *Brettanomyces sp*
- *Byssochlamys fulva*
- *Byssochlamys nivea*
- *Candida albicans*
- *Candida parapsilosis*
- *Clostridium perfringens*
- *Clostridium sporogenes*
- *Corynebacterium xerosis*
- *Dekkera naardenensis*
- *Enterococcus faecalis*
- *Gluconacetobacter liquefaciens*
- *Lactobacillus paracasei ssp. paracasei*
- *Lactobacillus plantarum ssp. plantarum*
- *Leuconostoc lactis*
- *Leuconostoc mesenteroides*
- *Malassezia furfur*
- *Neosartorya fischeri*
- *Penicillium expansum*
- *Penicillium paneum*
- *Saccharomyces cerevisiae*
- *Yarrowia lipolytica*
- *Zygosaccharomyces bailii*
- *Zygosaccharomyces rouxii*
for preserving compositions at a pH of about 1 to about 4, wherein said glycolipid represents an ustilagic acid or an ester of ustilagic acid esterified with a linear C₁-C₄ aliphatic alcohol.

In a preferred embodiment the said at least one ustilagic acid or its ester is added in a working amount of from about 1 to about 1,000 ppm - calculated on the composition.

In a preferred embodiment the said composition is a cosmetic, detergent or food preparation excluding beverages.

For the sake of good order, it is emphasized that all preferred embodiments mentioned above, particularly with respect to combinations, ratios and amounts also apply to the method and the use as claimed. Therefore, their repetition is not necessary.

### EXAMPLES

### MANUFACTURING EXAMPLES

### Fermentation

The strain *Ustilago maydis* 04507fxxx000001 was revived on 2% malt extract agar after storage at -80°C. As seed flasks 500ml-Erlemnmeyer flasks with 2 baffles and closed with PU-foam bungs were used. Each flask containing 100ml seed medium (malt extract 3%; Tween 85 0.2%; agar agar 0.1%; pH adjusted to pH 7.0) was inoculated with 3-4 agar pieces from the revival plates. The flasks were incubated for 48 hours at 25°C on an orbital shaker with 50mm shaking orbit. The main culture was inoculated at a ratio of 4% seed. For the main culture a fermenter of 12 L volume was filled with 9 L of a production medium containing dextrose 5%; ammonium sulfate 0.13%; potassiumdi-hydrogenphosphate 0.25‰; magnesium sulfate heptahydrate 0.13‰; sodium chloride 0.1‰; calcium chloride dihydrate 0.1‰; vitamin solution RPMI 1640 (Sigma-Al-drich) 1 ml/l and a trace element solution in tap water with the pH-value adhusted to pH 6.5. The trace element solution provided ferrous sulfate 5.4µM; zinc sulfate 3.1µM; manganese sulfate 2.4 µM; copper(II) sulfate 2.2 µM; cobalt nitrate 2.1µM; boric acid 4.0µM; and sodium molybdate 0.8µM. Fermentation conditions were incubation at 25°C, stirring at 350rpm with two Rushton type stirrers, aeration at 0.7vvm and foam control with a silicon based antifoam compound. After 120 hours of fermentation the culture was harvested by centrifugation. After centrifugation for 25 minutes with 10,000rcf biomass and ustilagic acid were sedimented.

### Extraction and Isolation of glycolipids

### Preparation of the raw extract

After harvest the ustilagic acids were contained in the centrifugation sediment together with the biomass from the fermentation. This sediment was frozen, lyophilized and extracted with warm ethanol. After evaporation of the solvent the raw extract was received.

### Pre-fractionation by reverse phase chromatography

The raw <extract was subjected to a reverse phase medium pressure chromatography to enrich the compounds of interest. Reverse phase MPLC allows to remove sugars and lipids as well as other secondary metabolites like flavonoids very efficiently and to reach enriched fractions of compounds of interest, which could be put on a second fractions step by HPLC using finer RP material. Sometimes it makes sense, to combine different MPLC prefractionations methods to get a more specific enrichment of selected saponins, e.g., by combination of RP18 and RP4 material.

### Second purification step by reverse phase chromatography

Pure compounds were isolated by reverse phase chromatography using different fractions from the first separation step by MPLC. To reach a purity of >90% per compound sometimes several repeated separations by HPLC were necessary. Promising fractions from the preparative HPLC runs detected by light scattering detection ELSD were analyzed by LCMS. The solvent of all fractions containing compounds of interest was removed in vacuum followed by a freeze-drying step. Isolated compounds were characterized by LCMS and 1D and 2D NMR spectroscopy.

### Isolated compounds

**Table 1: Isolated compounds**

| **ID** | **Compound** | **Structure** | **Formula** | **CAS** |
|---|---|---|---|---|
| NP-018245 | | 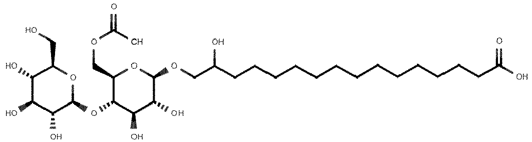 | C30H54O15 | 1427028-85-0 |
| NP-018428 | | 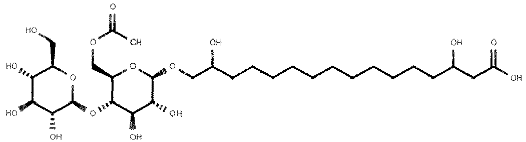 | C30H54O16 | 1198760-80-3 |
| NP-018243 | | 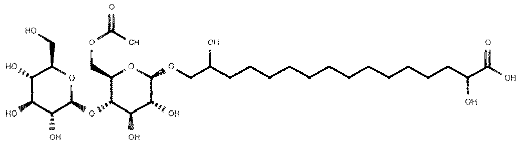 | C30H54O16 | 1427028-84-9 |
| NP-018247 | | 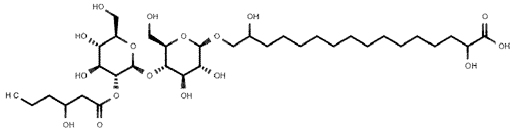 | C34H62O17 | |
| NP-018486 | 5 | 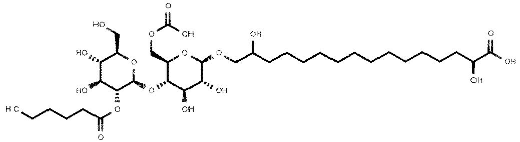 | C36H64O17 | |
| NP-018255 | | 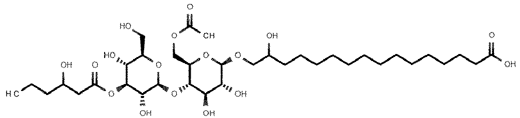 | C36H64O17 | 1427028-86-1 |
| NP-018250 | 3 | 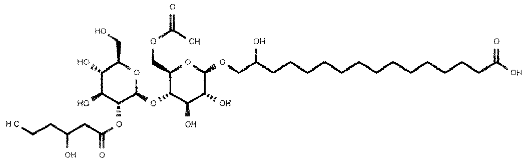 | C36H64O17 | 1427028-86-1 |
| NP-018756 | | 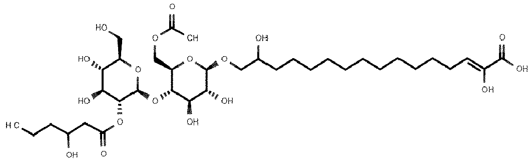 | C36H62O18 | |
| NP-018249 | | 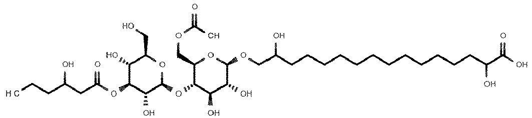 | C36H64O18 | 1427087-15-7 |
| NP-018459 | | 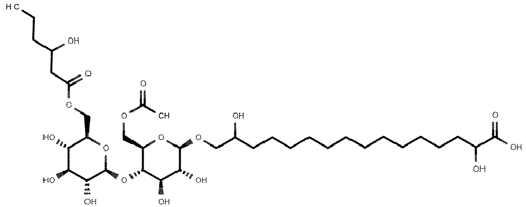 | C36H64O18 | 959757-52-9 |
| NP-018217 | 1 | 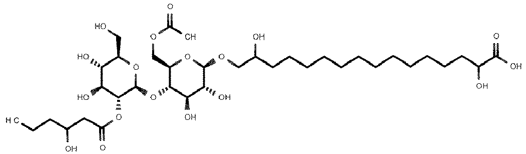 | C36H64O18 | 867578-35-6 |
| NP-018752 | 4 | 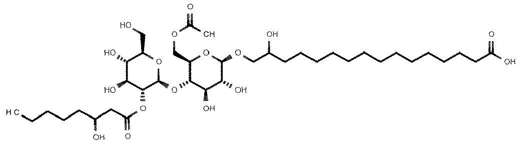 | C38H68O17 | |
| NP-018753 | 6 | 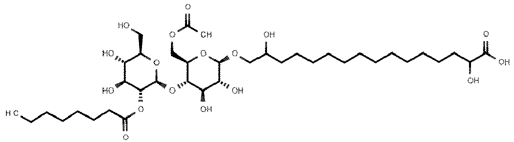 | C38H68O17 | 1427039-33-5 |
| NP-018218 | | 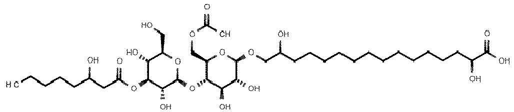 | C38H68O18 | 1427087-13-5 |
| NP-018256 | 2 | 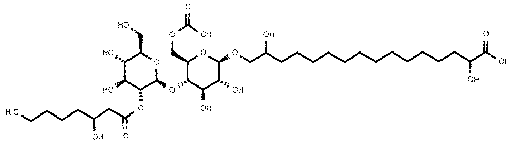 | C38H68O18 | 1427087-16-8 |
| NP-018430 | | 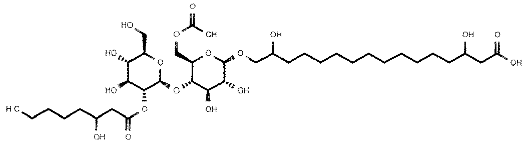 | C38H68O18 | 1199256-46-6 |
| NP-018432 | | 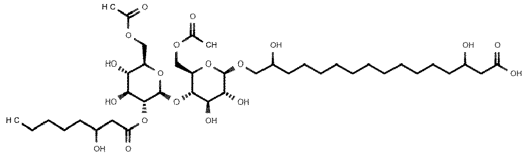 | C40H70O19 | 1427113-19-6 |
| NP-018431 | | 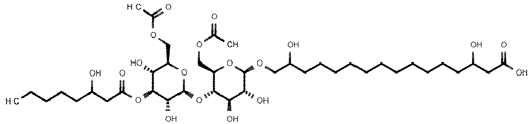 | C40H70O19 | |

### Antimicrobial activity

Compounds are dissolved at a concentration of 20mg/mL using 100% DMSO.
- Preculture: yeast and bacteria are plated on potato dextrose agar, resuspended in potato dextrose broth and CFU via OD measurement determined; adjusting CFU of the strain in PDB pH 3.8
- Preculture: for mycel forming fungi spores were directly used after thawing of cryopreserved stocks of known titre
- Final titre used for the assay: Yeast strains 5*10³ CFU/ml, Bacteria 1*10⁶ CFU/ml, Fungi (spores) 5*10⁴ CFU/ml
- Potato dextrose broth pH 3.8 was used as assay matrix
- 500ppm (0.05%) of the compounds were screened (final DMSO 2.5% (v/v))
- Volume: 100µl in 96-well format
- Positive controls: potassium sorbate, sodium benzoate & triclosan
- 28°C to 30°C, static incubation
- Incubation time depending on spoilage organism between 2 and 8 days
- Visual evaluation (per eye, for fungi with binocular magnification)

**Table 2: List of strains**

| **Class** | *Taxon* | *Strain no.* |
|---|---|---|
| **yeast** | *Saccharomyces cerevisiae* | DSM 2548 |
| | *Zygosaccharomyces bailii* | *SUN02* |
| | Candida albicans | ATCC10231 |
| | *Candida parapsilosis* | *SUN05* |
| | *Saccharomyces cerevisiae* | *DSM 2548* |
| | *Zygosaccharomyces bailii* | *DSM 70834* |
| | *Brettanomyces sp* | *SUN08* |
| | *Brettanomyces naardensis* | *CBS 6115* |
| | *Zygosaccharomyces rouxii* | *DSM 7525* |
| | *Yarrowia lipolytica* | *DSM 70561* |

| **bacteria** | *Asaia siamensis* | *SUN03* |
|---|---|---|
| | *Alicyclobacillus acidoterrestris* | DSM 2498 |
| | Bacillus licheniformis | DSM 13 |
| | Bacillus subtilis | ATCC 6633 |
| | Clostridium perfringens | DSM756 |
| | Clostridium sporogenes | ATCC19404 |
| | Corynebacterium xerosis | DSM 20743 |
| | Enterococcus faecalis | ATCC 7080 |
| | *Gluconacetobacter liquefaciens* | DSM 5603 |
| | *Lactiptantibacittus plantarum ssp. plantarum* | DSM 20174 |
| | Lactobacillus paracasei ssp. paracasei | DSM 46331 |
| | Leuconostoc lactis | DSM 20202 |
| | Leuconostoc mesenteroides | DSM 20343 |

| **fungi** | *Penicillium paneum* | SUN04 |
|---|---|---|
| | *Neosartorya fisheri* | SUN09 |
| | *Byssochlamys nivea* | SUN10 |
| | Byssochlamys fulva | DSM 1808 |
| | *Aspergillus brasiliensis* | DSM 1988 |
| | *Penicillium expansum* | DSM 62841 |
| | *Malassezia furfur* | DSM 6170 |
| | Dekkera naardenensis | CBS 6115 |

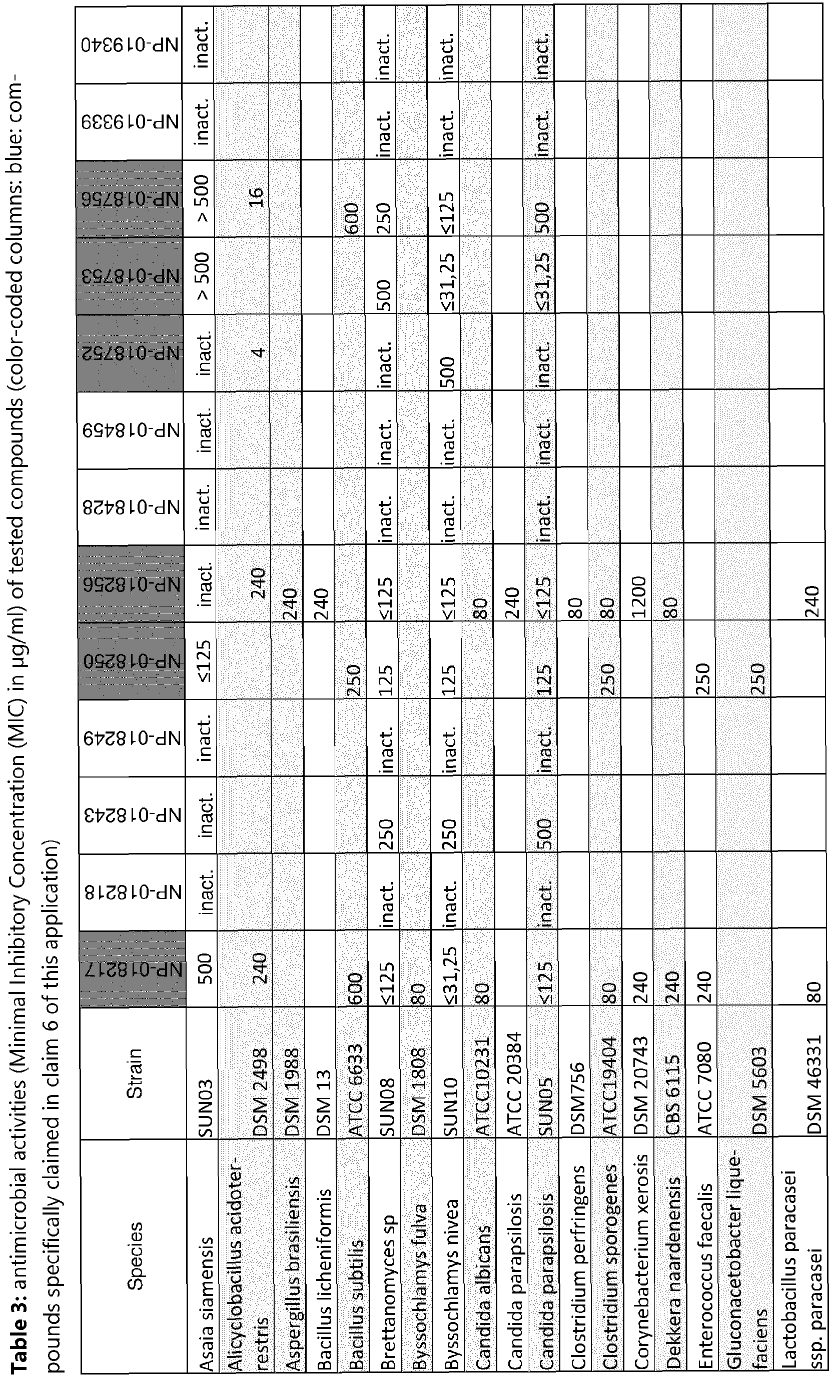

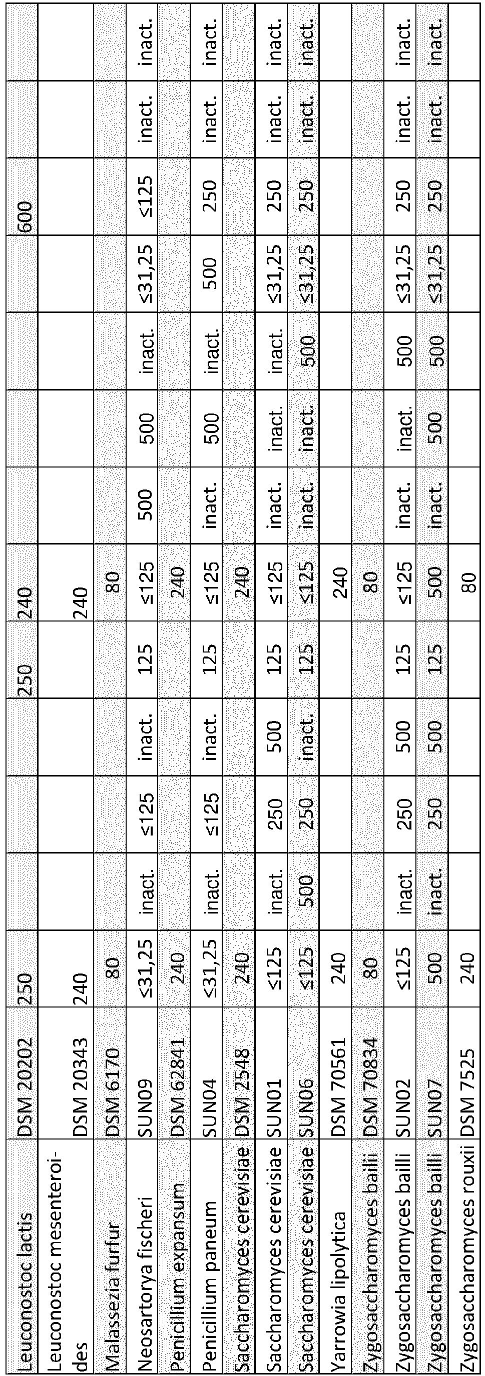

The examples clearly demonstrate that the glycolipids according to the present invention exhibit a serious activity against a wide spectrum of microorganisms, which typically can be found in products of daily life as foodstuff, petfood or cosmetics. The performance of glycolipids according to the present invention is comparable to sorbic and benzoic acid, common natural preservatives, and can be synergistically increased either by blending two or more glycolipids according to the invention or by using blends of said glycolipids with secondary preservatives like sorbic acid, benzoic acid, lactic acid, ethanol or PHB (esters).

### FORMULATION EXAMPLES

### FORMULATION EXAMPLES FOOD

The following examples **F1 to F2** show various formulations for food preparations. Glycolipid stands for one or more glycolipids according to Claim 4.

### EXAMPLE F1

An Italian Salad dressing formulation is prepared as follows:

| Ingredient | Parts |
|---|---|
| Part I | |
| water | 70.0 |
| Vinegar | 7.0 |
| Sodium Chloride | 2.0 |
| Sweetener | 4.0 |
| Gum | 0.4 |
| Flavors | 0.9 |

| Part II | |
|---|---|
| Vegetable Oil | 5.3 |
| Part III | |
| Water | 6.2 |
| Vinegar | 4.2 |
| Spices and Flavors | 1.8 |

Parts 1 and 2 are mixed and homogenized. Part 3 is then added and thoroughly mixed to form a homogenous mixture. 400 ppm of glycolipid is added to the dressing formula.

### EXAMPLE F2: Ketchup

**aqueous phase**

| | |
|---|---|
| ground tomato | 25.9 |
| cider vinegar | 20.7 |
| water | 44.3 |
| Sundried tomatoes | 2.1 |
| sodium chloride | 1.8 |
| dehydrated minced garlic | 1.1 |
| frozen oregano | 0.4 |
| basil | 0.5 |
| Xanthan gum | 0.2 |
| white onion powder | 2.5 |
| sucrose | 7.0 |
| EDTA | 0.0007 |
| glycolipid | 0.05 |

| **oil phase** | |
|---|---|
| soybean oil | 53.3 |
| olive oil | 46.7 |

### FORMULATION EXAMPLES PETFOOD

The following examples **P1 to P4** show various formulations for pet food preparations. Glycolipid stands for one or more glycolipids according to Claim 4.

### EXAMPLE P1

This example describes the preparation of a 60% moisture, microbiologically-stable food product simulating meat chunks which is suitable for use as a dog food. The food is prepared from the following ingredients:

| Ingredient | Parts |
|---|---|
| Wheat gluten | 10.0 |
| Soya bean oil meal | 4.0 |
| Poultry meal | 3.0 |
| Meat meal | 1.5 |
| Corn meal | 1.2 |
| Tallow | 5.0 |
| Carrageenan | 2.5 |
| Potassium sorbate | 0.3 |
| Tween 80 | 0.25 |
| Span 80 | 0.75 |
| Glycolipid | 0.5 |
| High fructose corn | 15.0 |
| syrup (80% solids, | |
| 90% fructose) | |
| Water | 56.0 |

To prepare the food, the carrageenan is added to the water at 70° C. and stirred to dissolve. The Tween 80, polyoxyethylene sorbitan monoleate available from Atlas Chemical, potassium sorbate, benzyl alcohol and the high fructose corn syrup (Isomerose 900, available from Clinton Corn Products) are added to the carrageenan solution and stirred vigorously to dissolve. The tallow is melted and the Span 80, sorbitan monooleate, available from Atlas Chemical, is added to it and mixed thoroughly. The tallow and aqueous solution are then mixed vigorously to emulsify. The remaining dry components are added with stirring to blend with the emulsion. The finished mix is added to a pan and autoclaved at 15 lbs gauge pressure for 20 minutes to heat set the mass. The heat-set cohesive product has a moisture content of 60% and is microbiologically stable. Without the glycolipid component this formulation would not be microbiologically stable.

### EXAMPLE P2

This example describes the production of a 70% moisture, microbiologically stable food product simulating meat chunks from the following ingredients:

| Ingredient | Parts |
|---|---|
| Wheat gluten | 7.48 |
| Soya bean oil meal | 2.96 |
| Poultry meal | 2.23 |
| Meat meal | 1.11 |
| Corn meal | 0.88 |
| Tallow | 3.70 |
| Carrageenan | 1.85 |
| Potassium sorbate | 0.30 |
| Span 80 | 0.75 |
| Tween 80 | 0.25 |
| Glycolipid | 0.50 |
| High fructose corn syrup | 10.10 |
| Water | 66.90 |

The process is the same as in Example 1.

### EXAMPLE P3

This example describes the preparation of a microbiologically-stable gravy having moisture content of 60%. The formulation is as follows:

| Ingredient | Parts |
|---|---|
| Meat & bone meal | 9.45 |
| Yeast | 1.35 |
| Corn flour | 1.35 |
| Dried whey | 1.35 |
| Whole egg solids | 1.35 |
| Tallow | 9.45 |
| High fructose corn syrup | 20.23 |
| Tween 80 | 0.25 |
| Span 80 | 0.75 |
| Glycolipid | 0.50 |
| Kelcosol | 0.14 |
| Water | 54.0 |

The process according to this example calls for first mixing the Span 80 with the tallow in melted condition. Next, a solution containing the high fructose corn syrup, water, potassium sorbate, Tween 80, kelcosol, sodium alginate, available from Kelco Co., and benzyl alcohol is prepared by heating the water to 60° C. and mixing. The solution is then stirred vigorously with the tallow to emulsify. The remaining dry materials are then added and stirred vigorously. No heating is necessary other than in the initial mixing of the fat and aqueous phases.

### EXAMPLE P4

This example describes the preparation of a microbiologically-stable gravy having a moisture content of 70%. The following ingredients are combined according to the process of Example 3:

| Ingredient | Parts |
|---|---|
| Meat & bone meal | 7.0 |
| Corn flour | 1.0 |
| Brewers yeast | 1.0 |
| Dried whey | 1.0 |
| Whole egg solids | 1.0 |
| Tallow | 7.0 |
| High fructose corn syrup | 15.0 |
| Kelcosol | 0.1 |
| Span 80 | 0.75 |
| Tween 80 | 0.25 |
| Glycolipid | 0.50 |
| Water | 65.30 |

### FORMULATION EXAMPLES COSMETICS & DETERGENTS

The following examples **F1 to F56** show various formulations for preparations. Glycolipid stands for one or more glycolipids according to Claim 4.

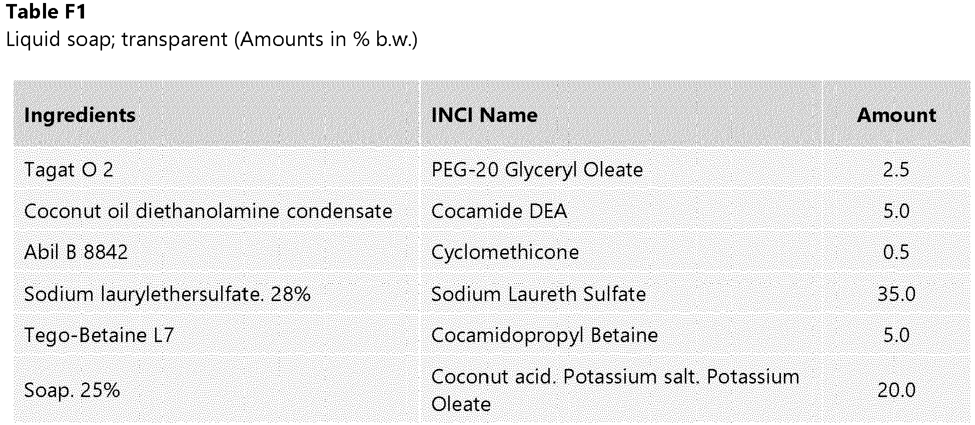

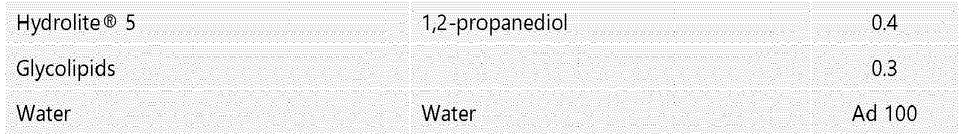

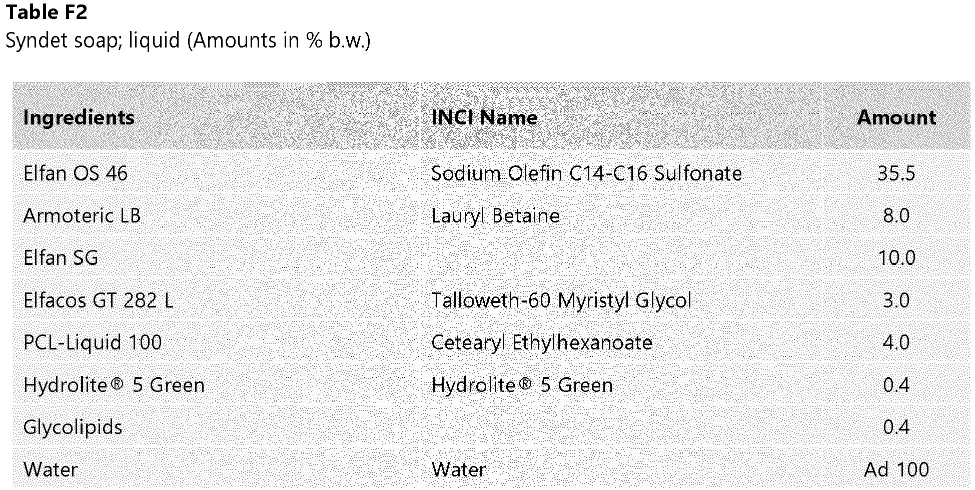

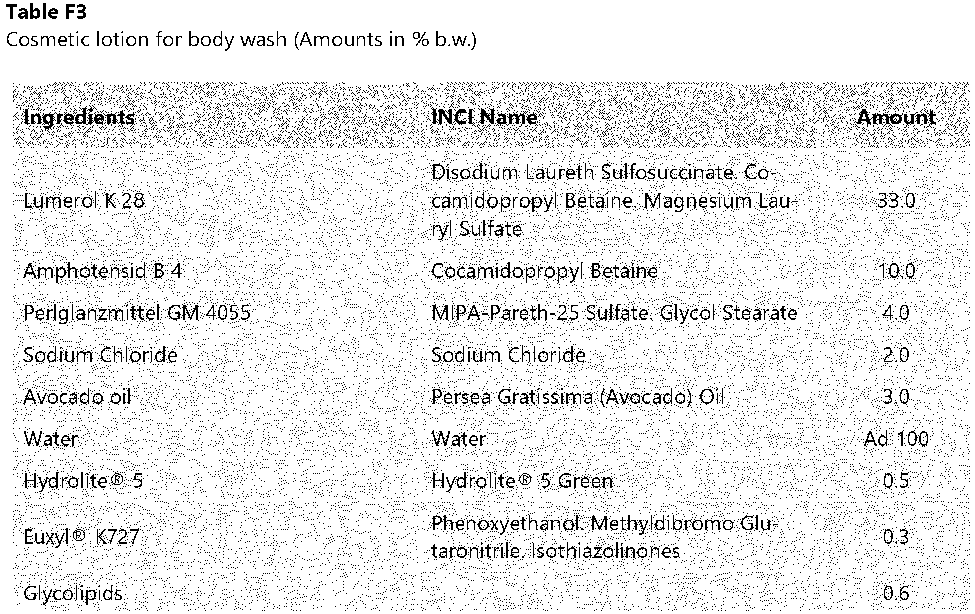

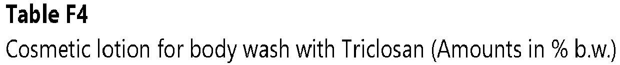

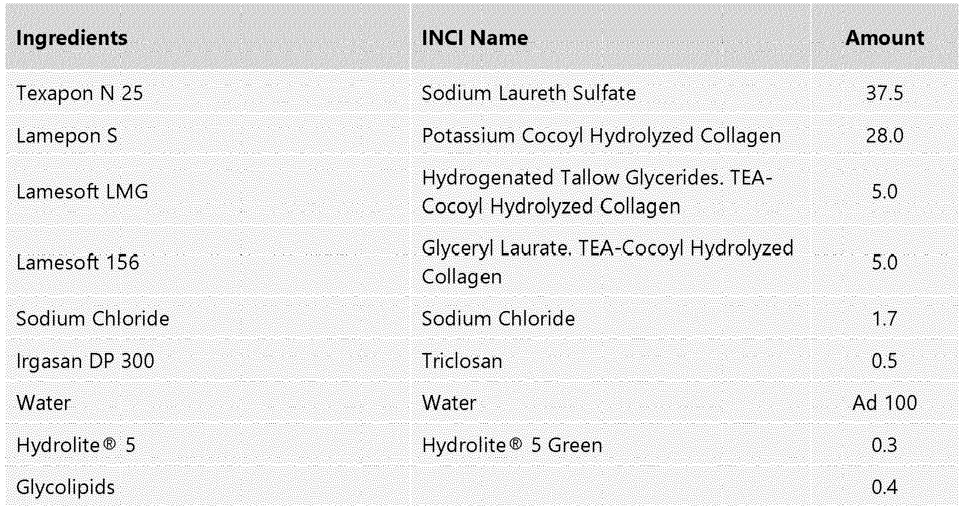

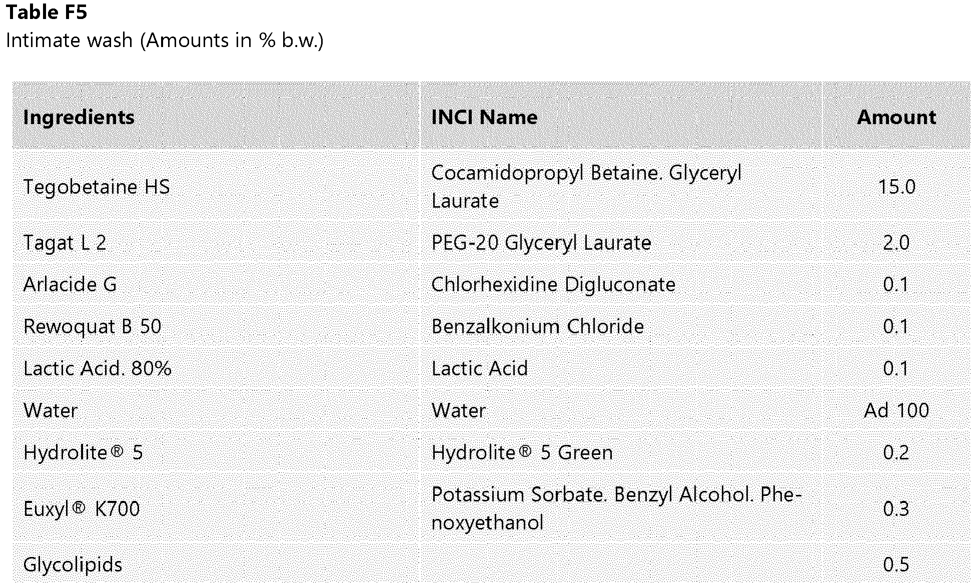

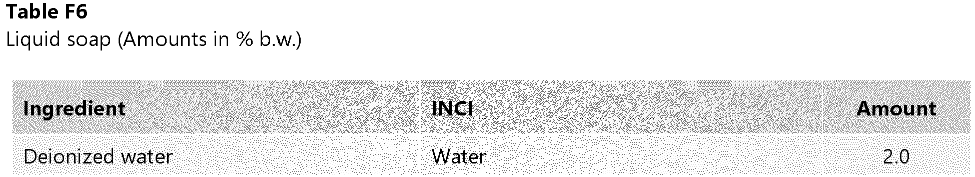

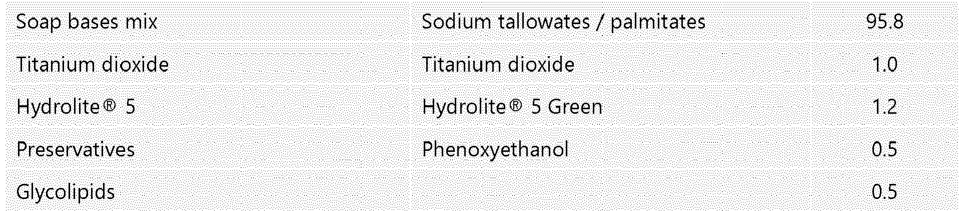

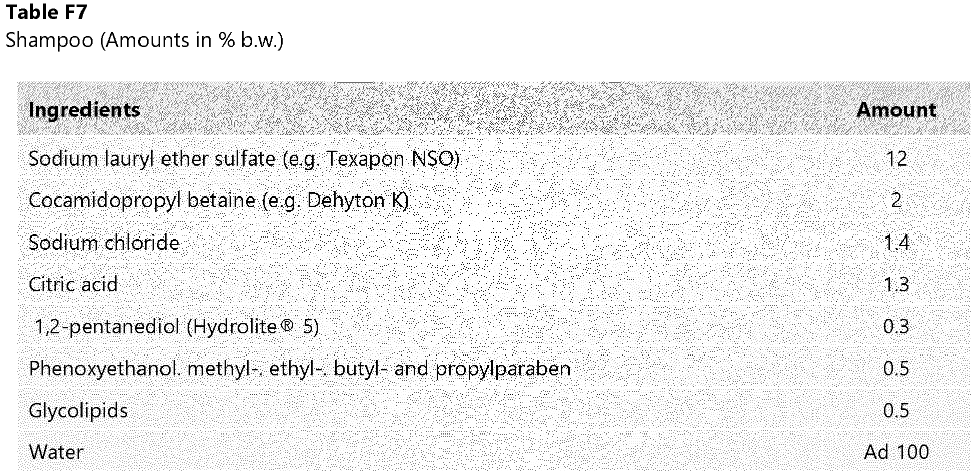

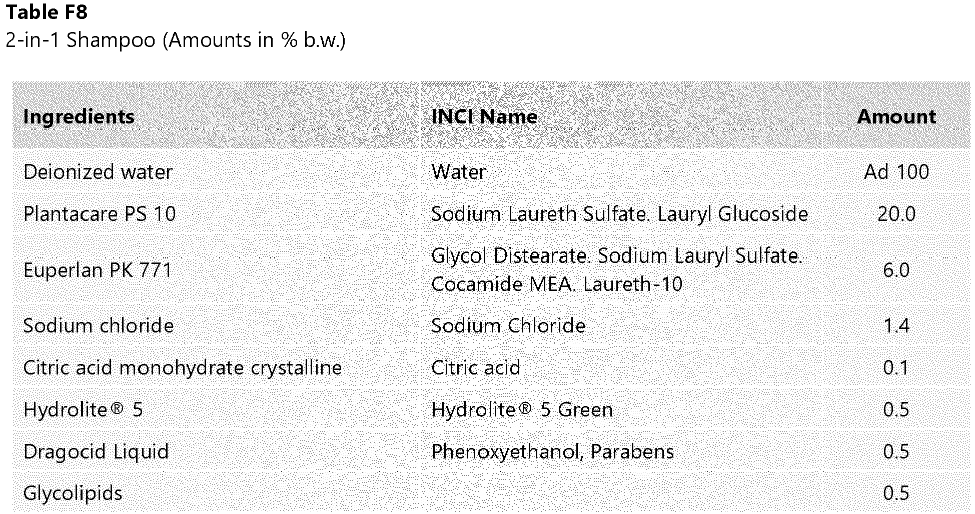

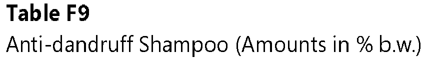

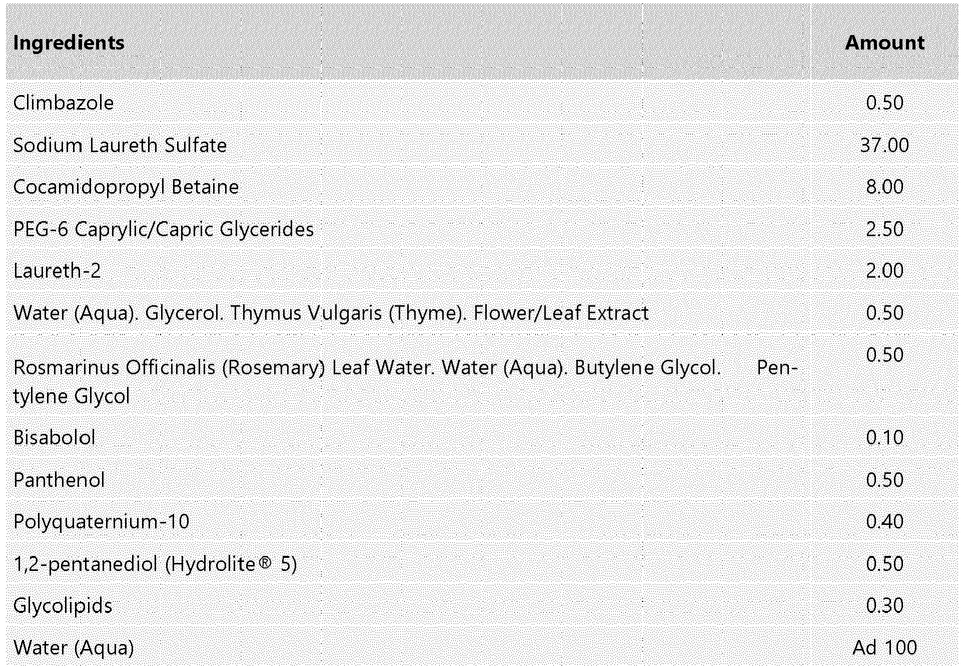

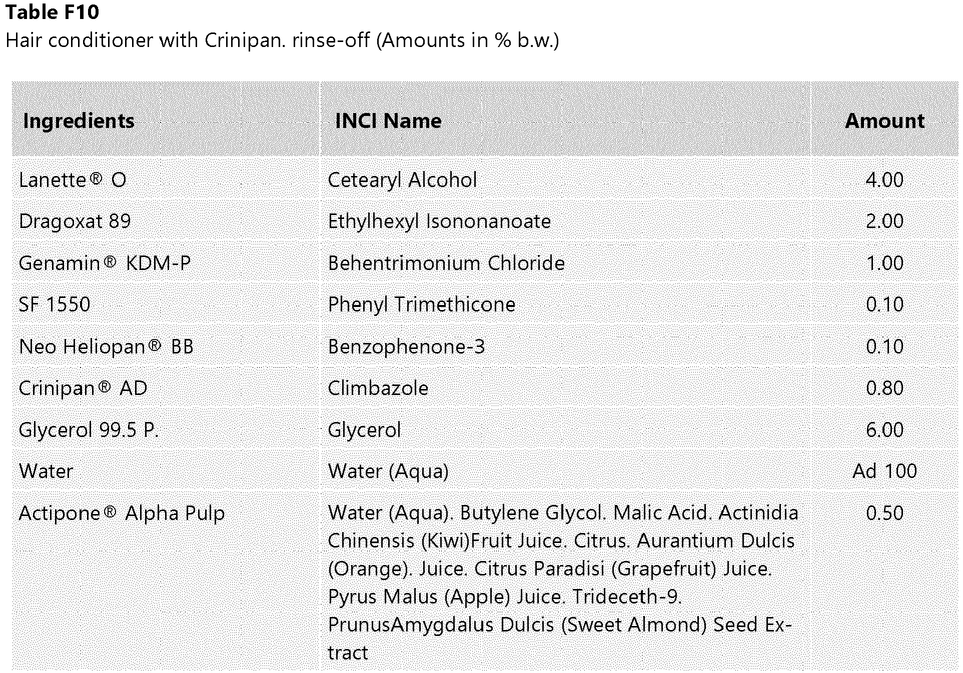

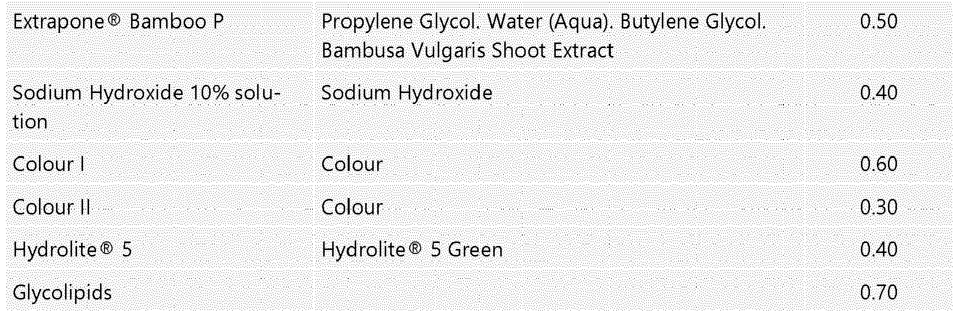

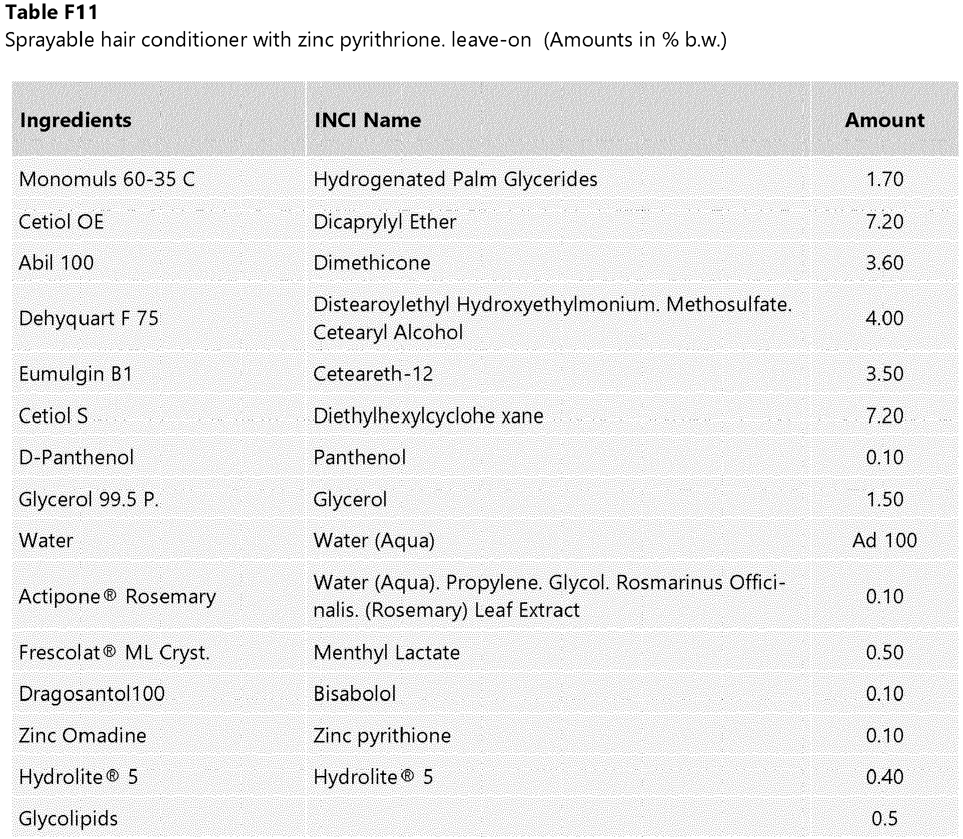

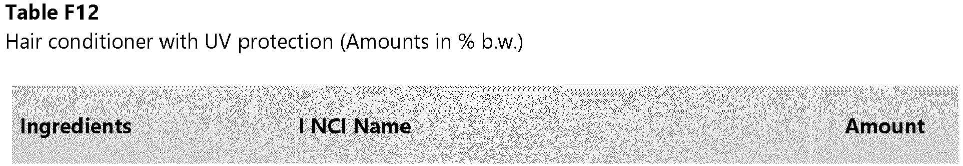

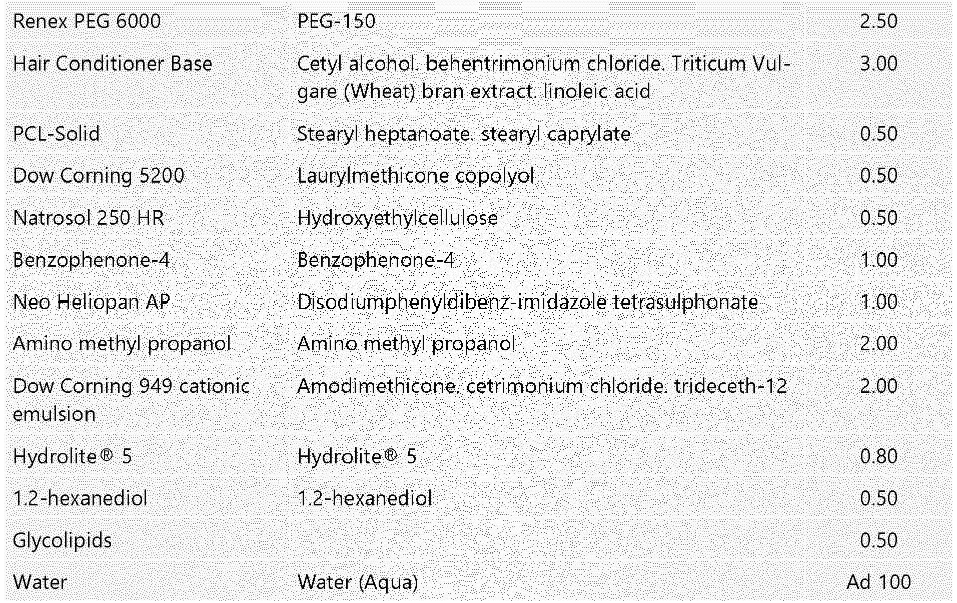

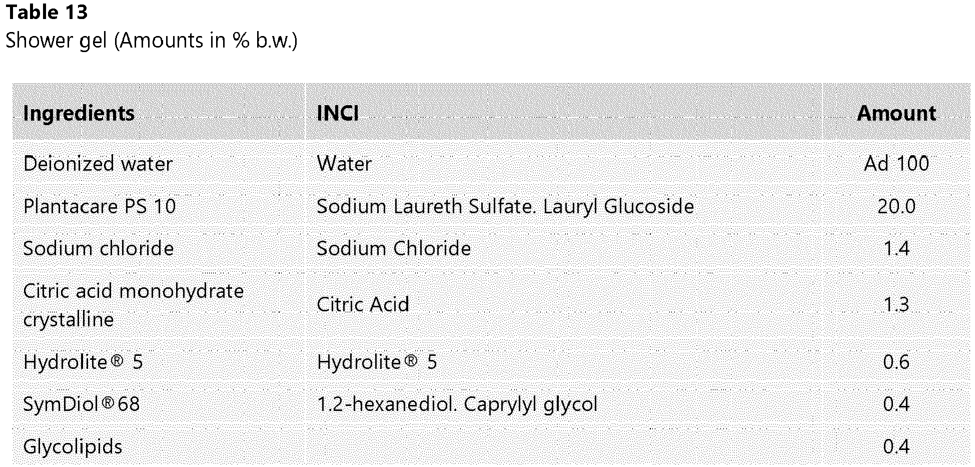

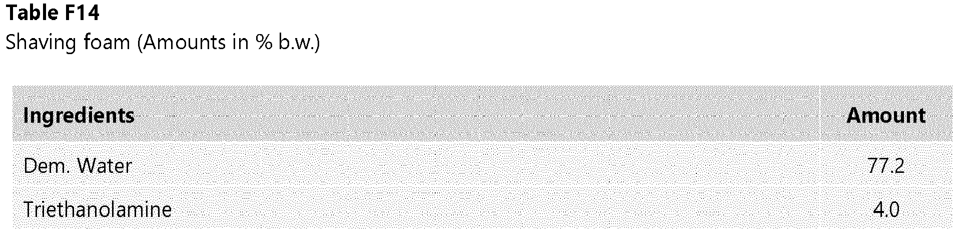

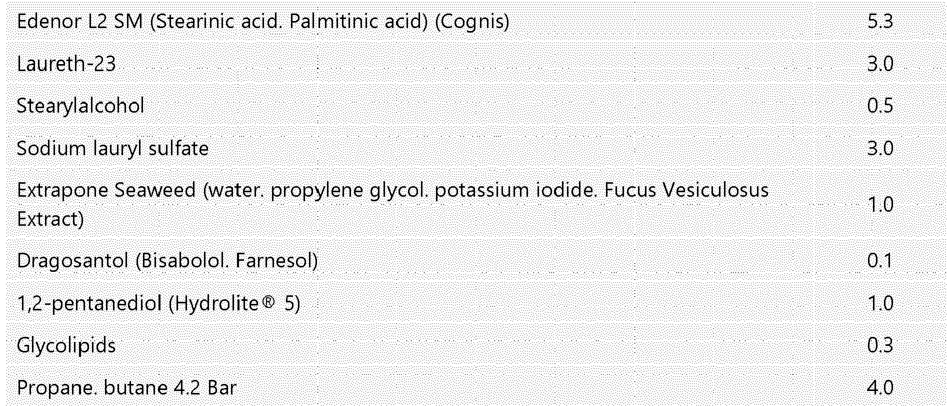

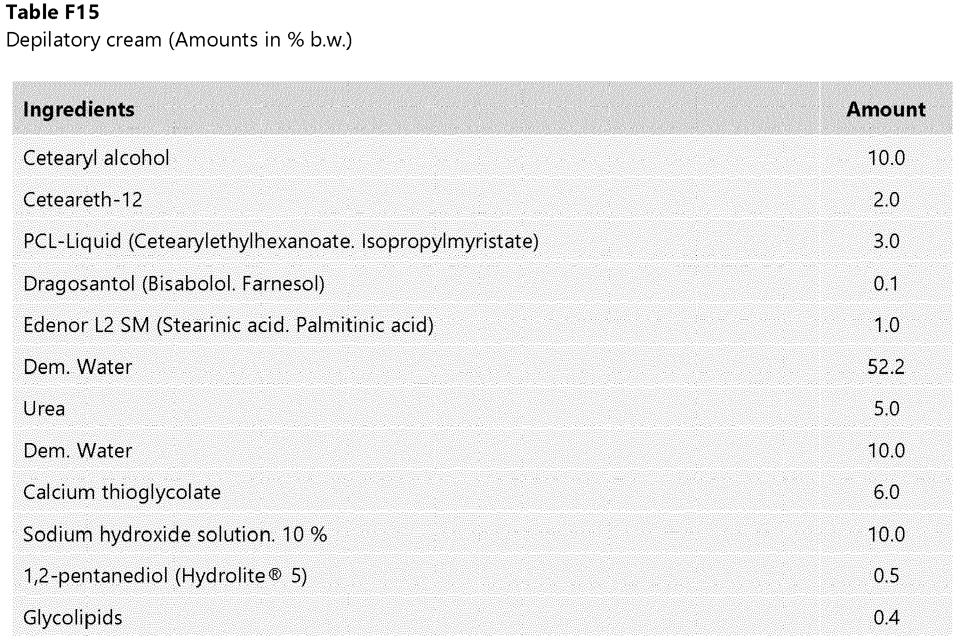

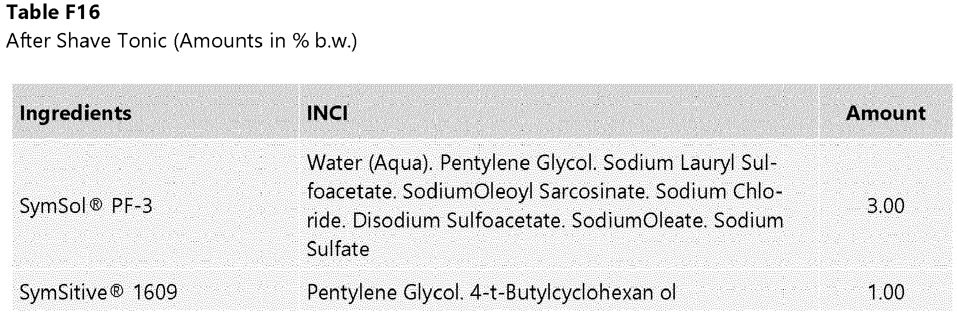

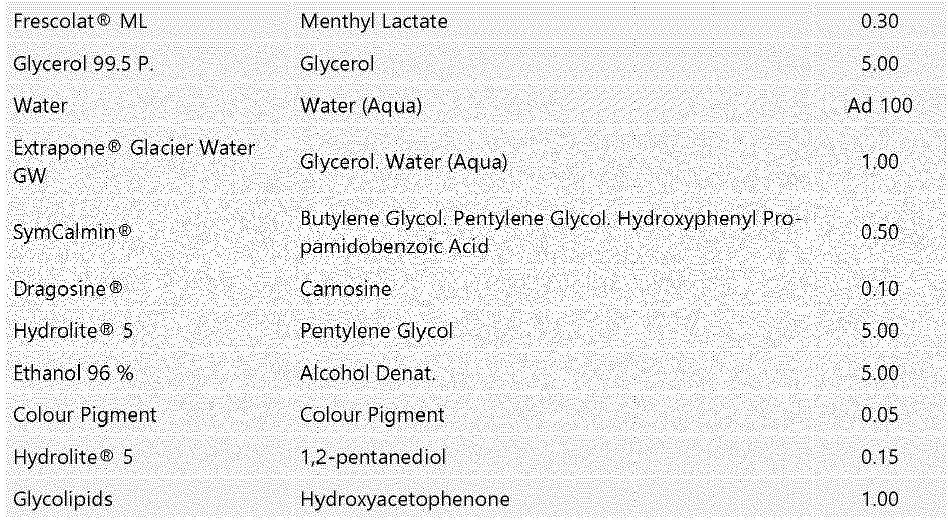

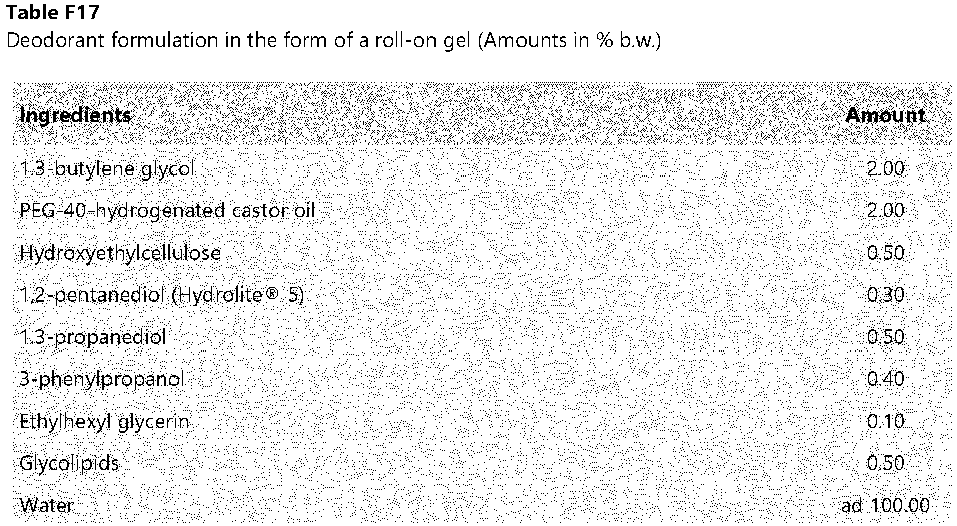

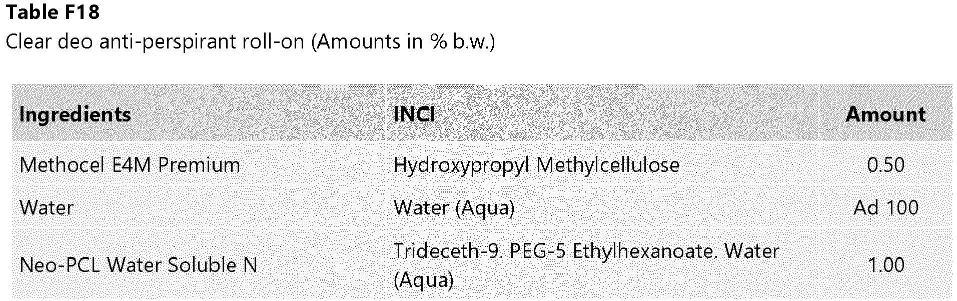

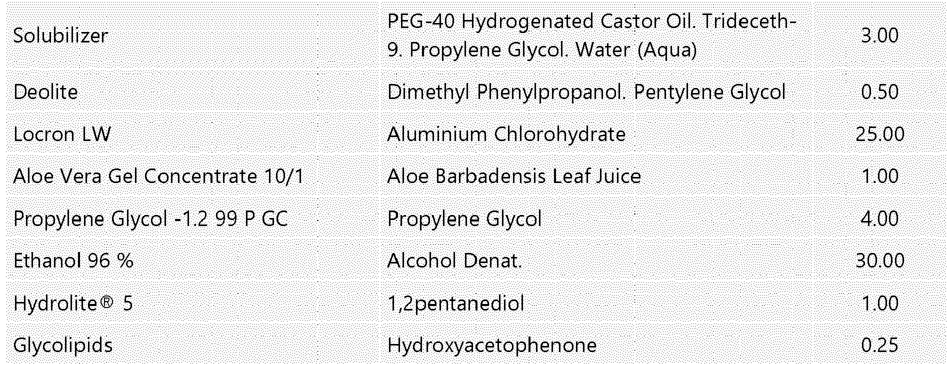

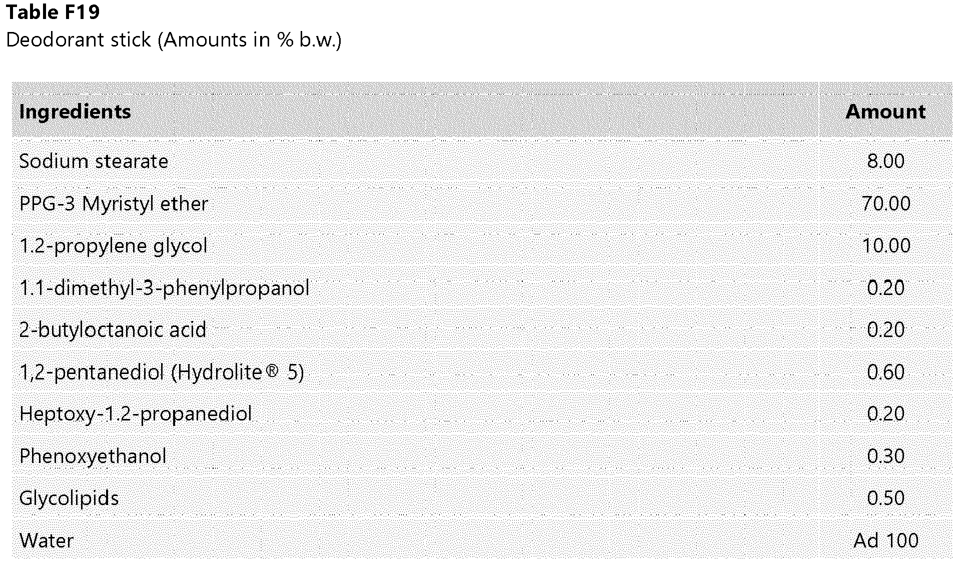

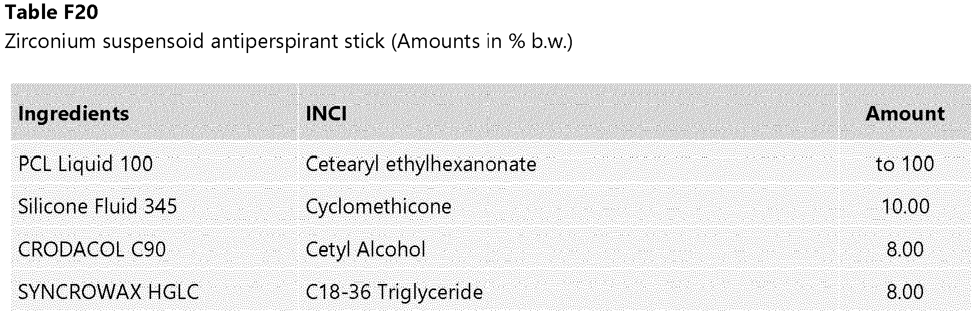

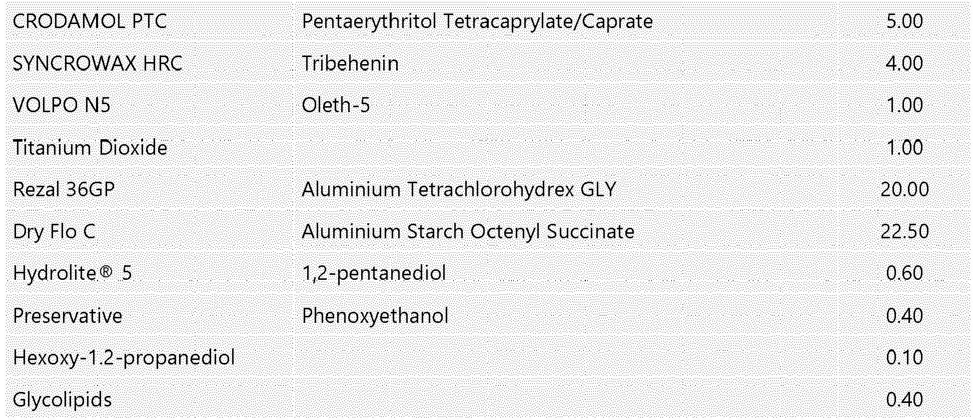

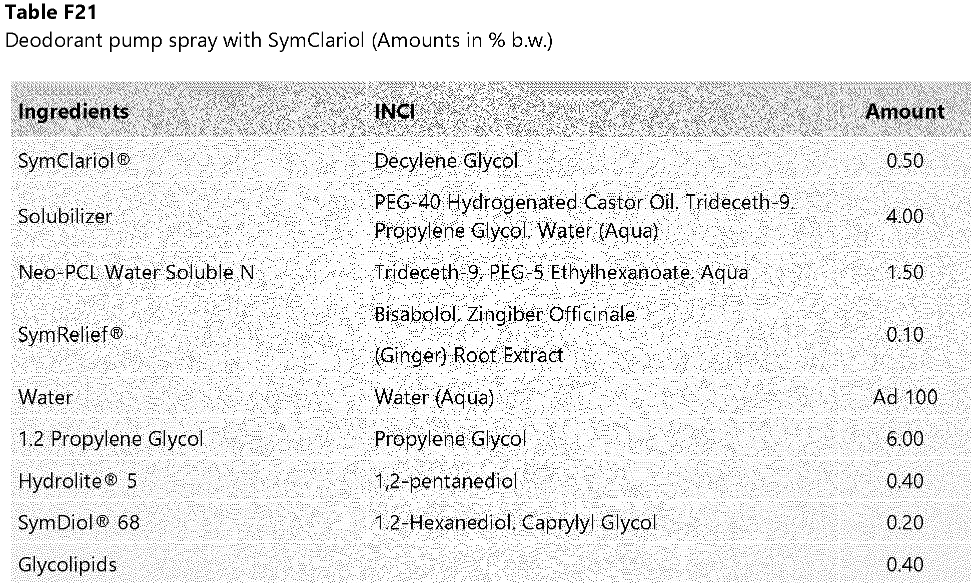

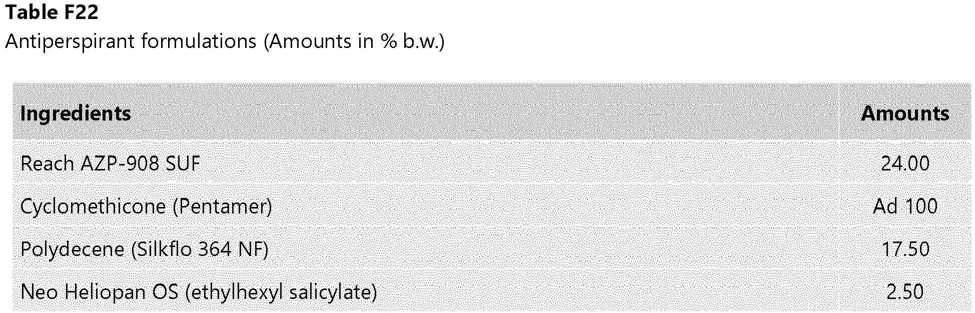

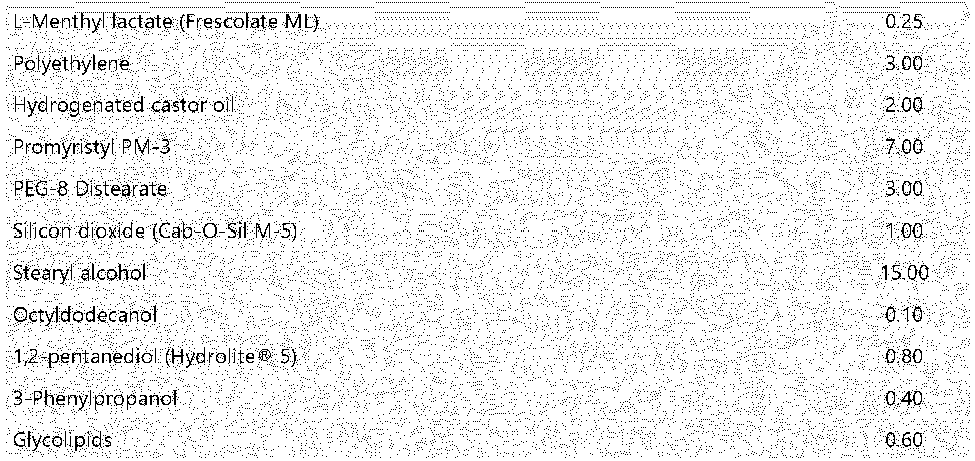

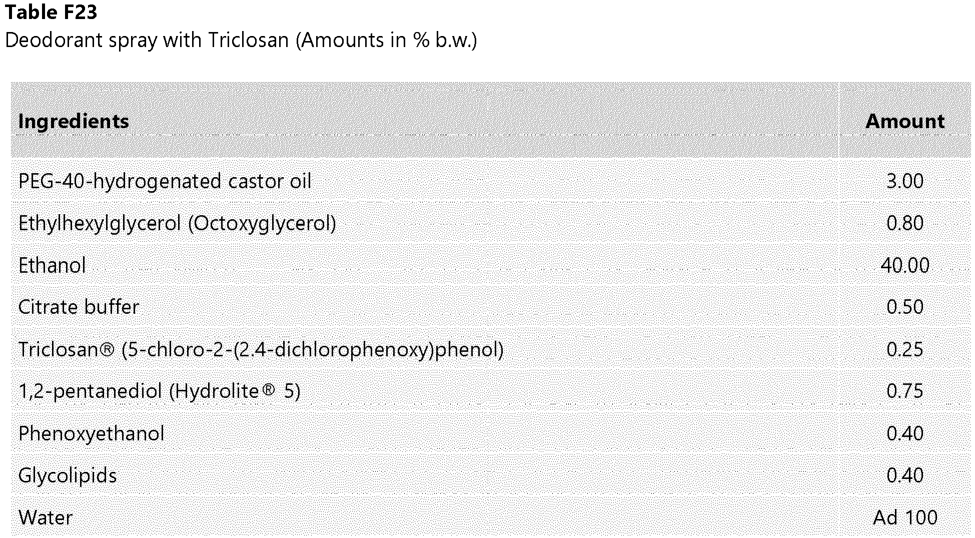

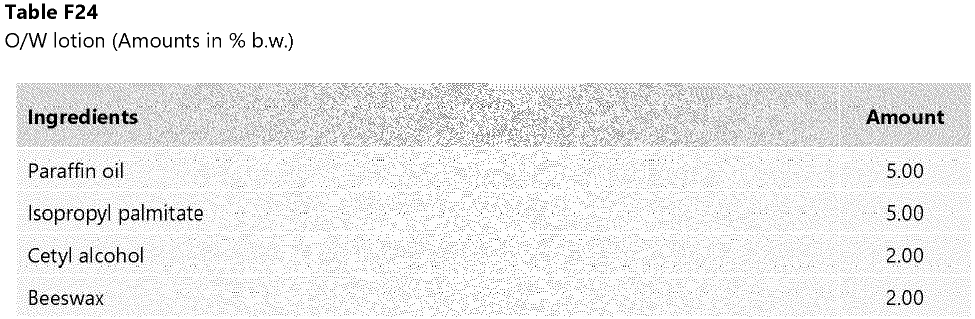

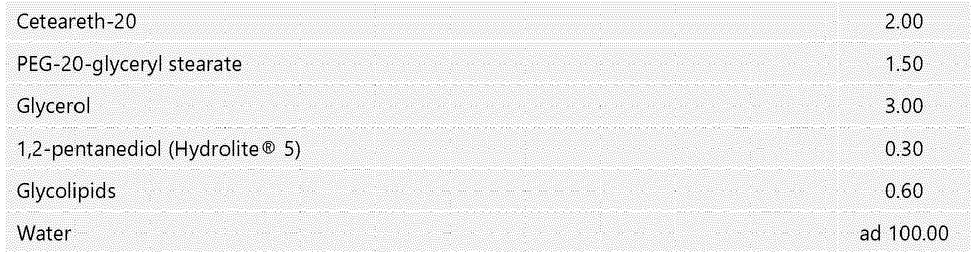

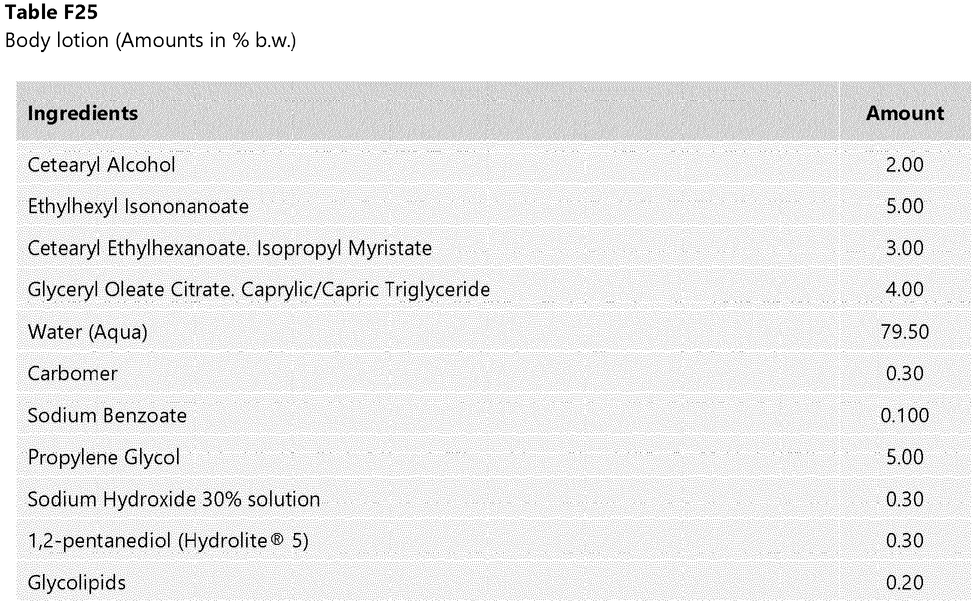

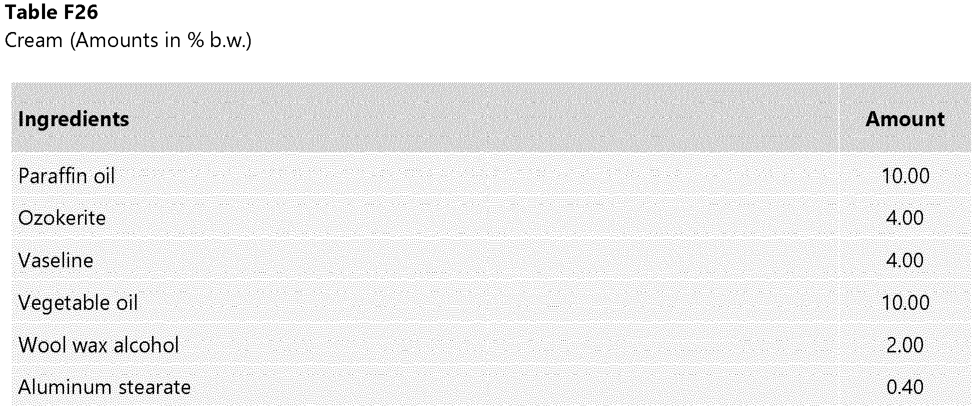

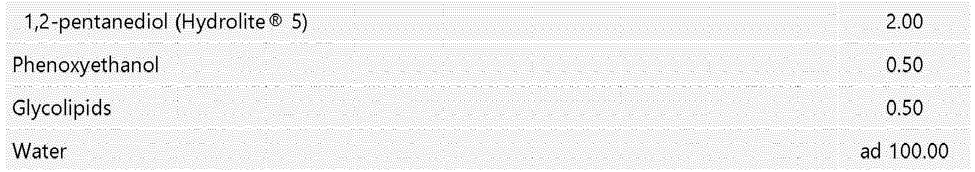

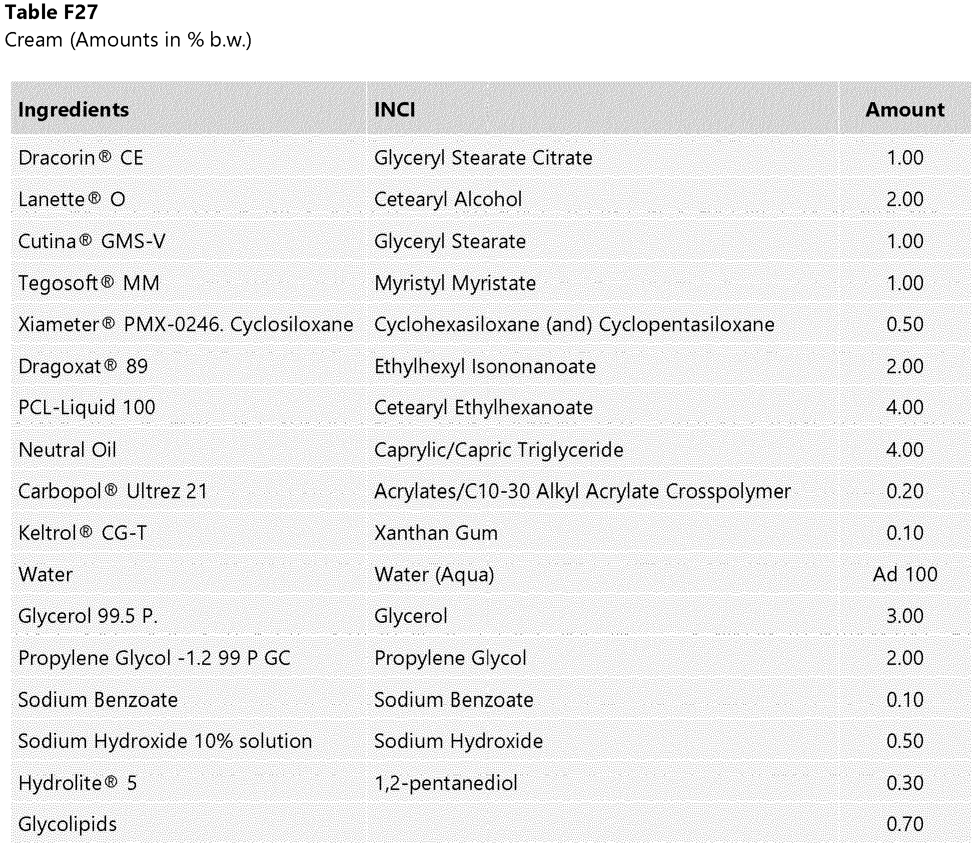

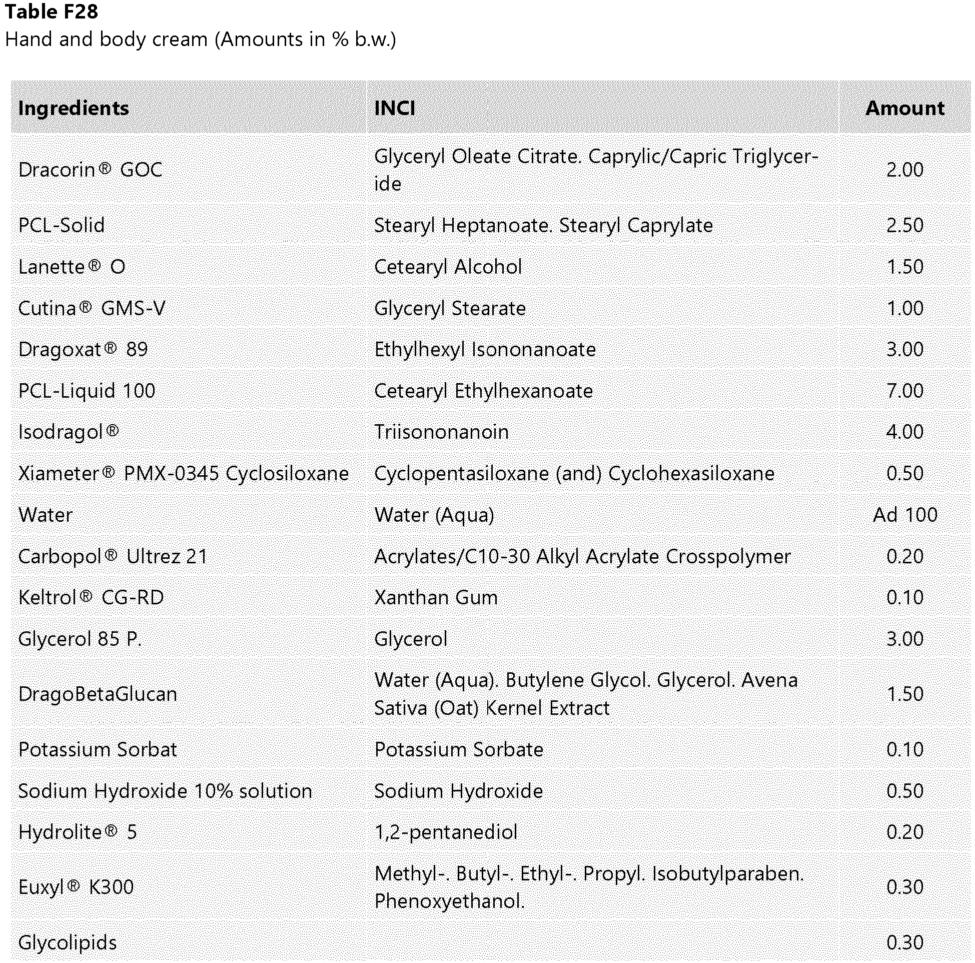

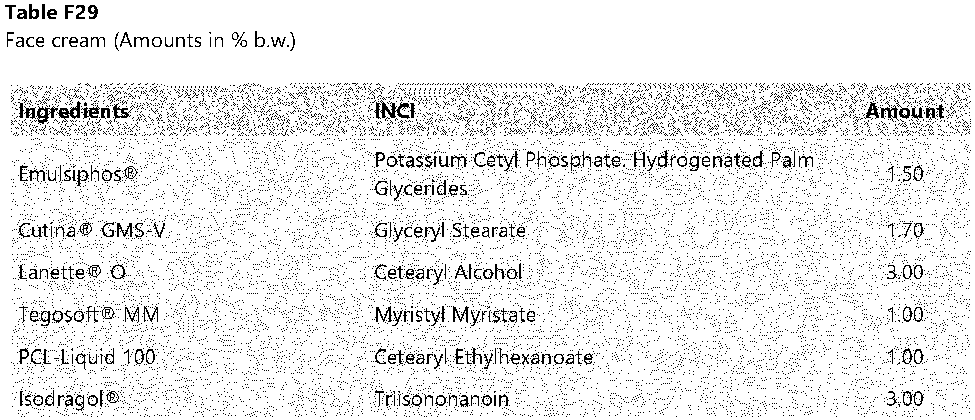

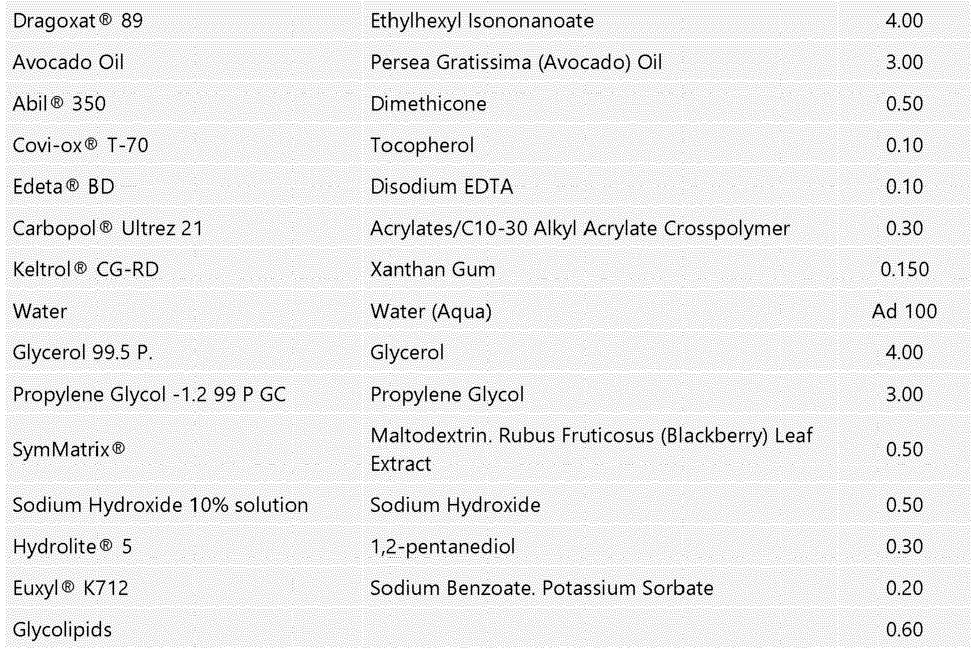

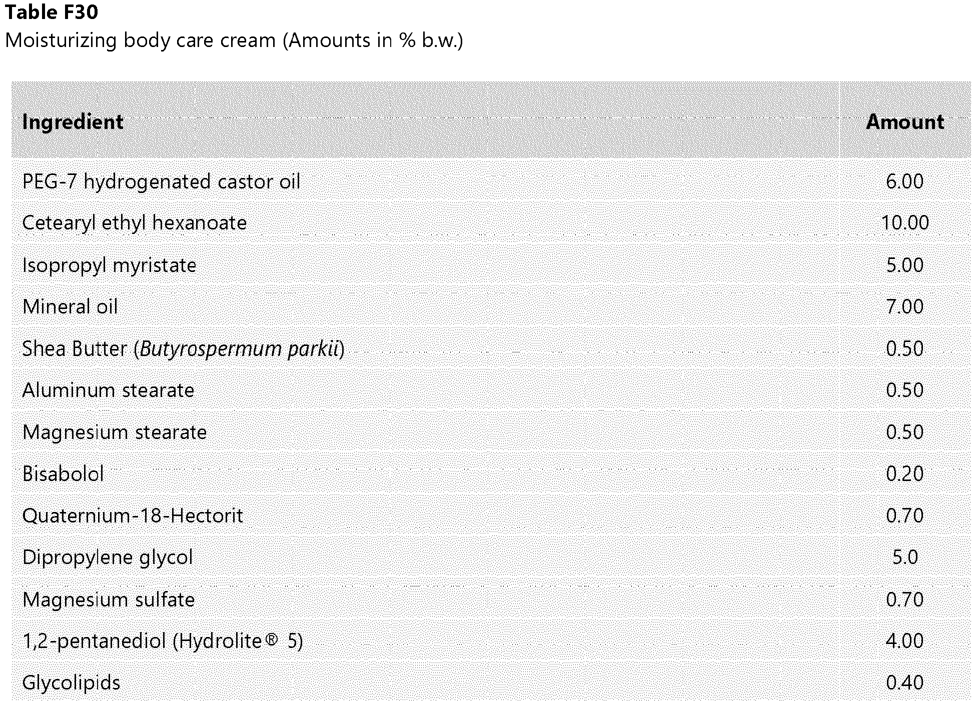

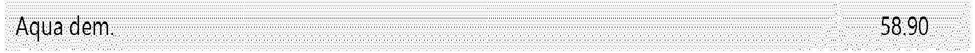

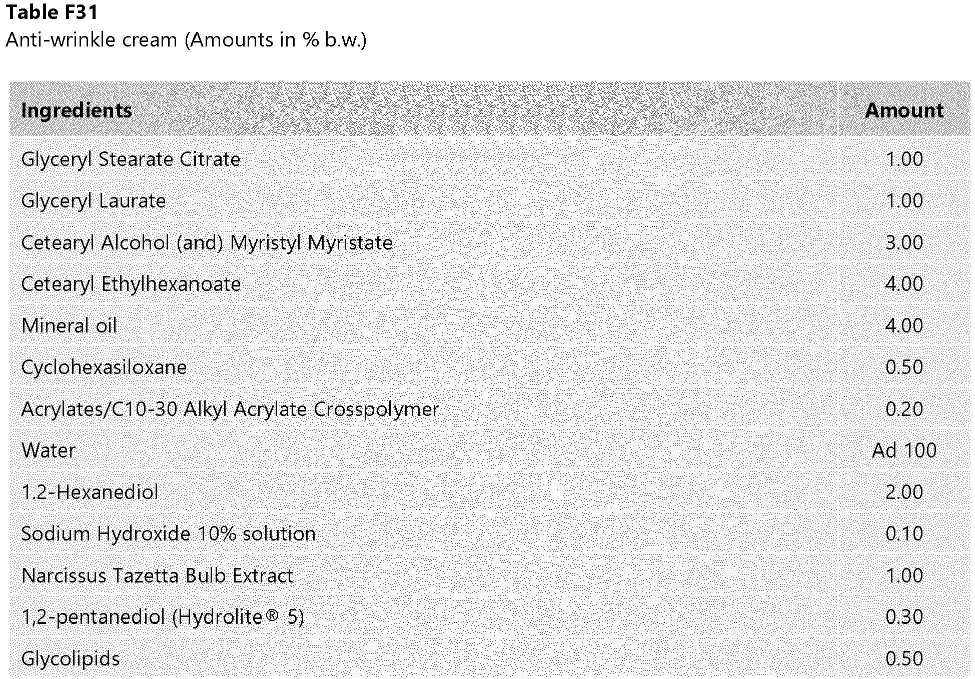

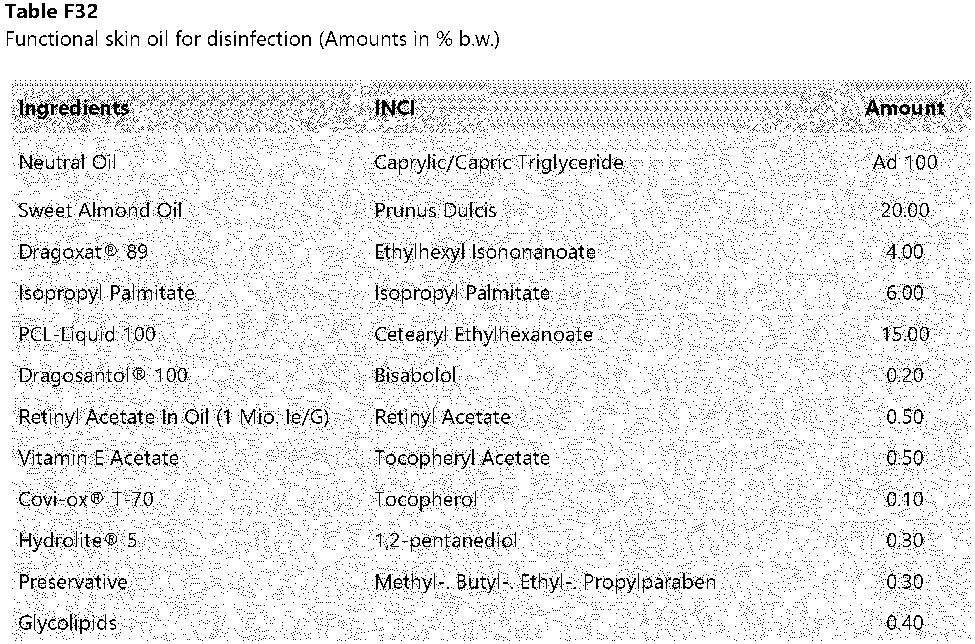

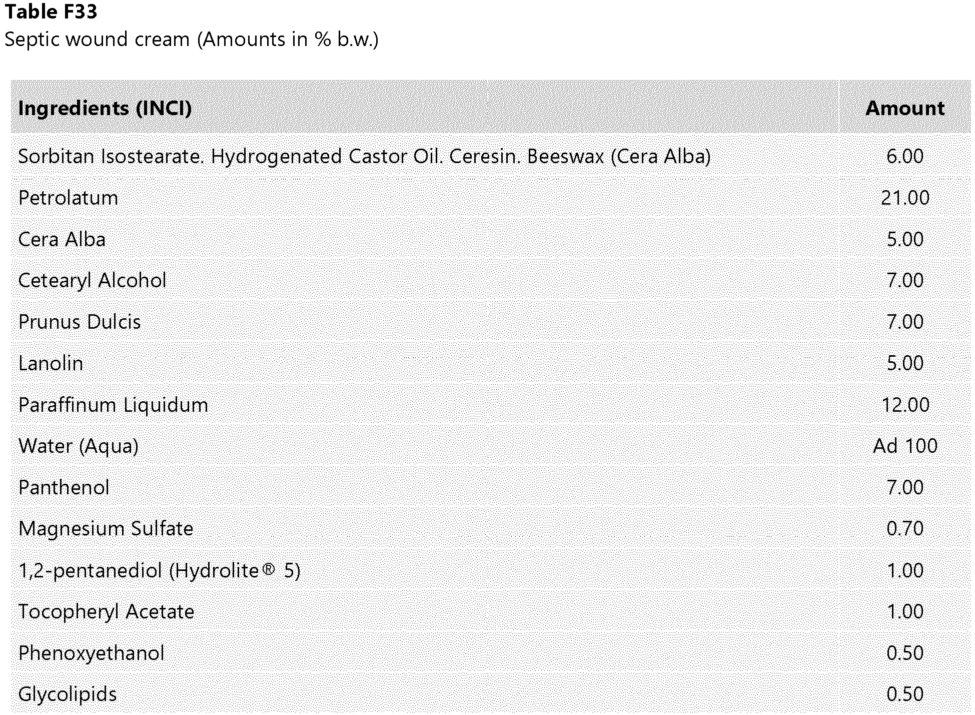

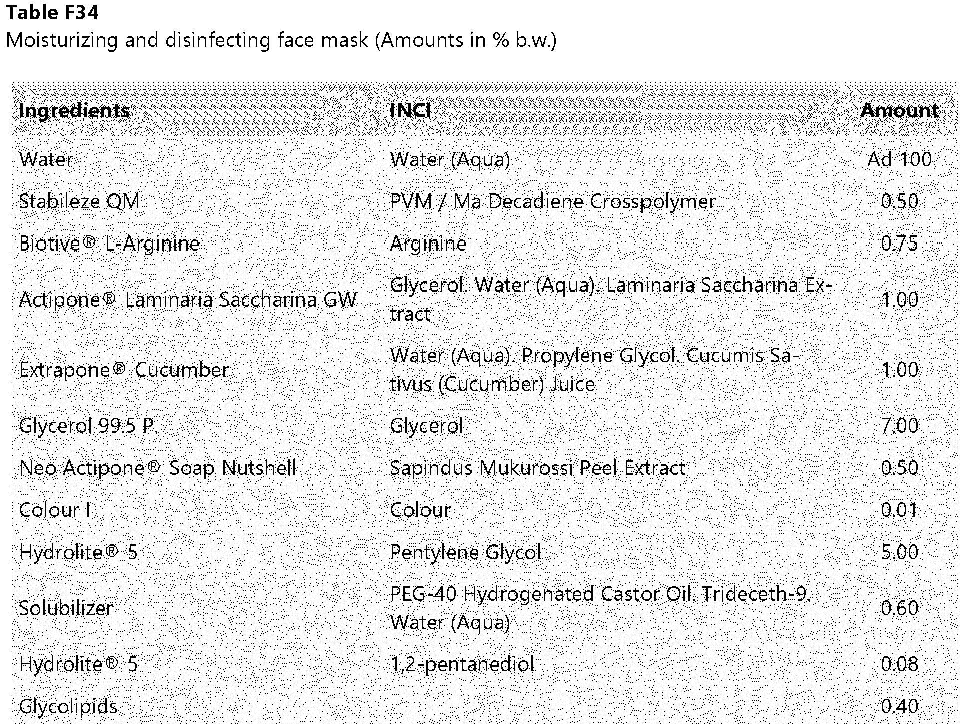

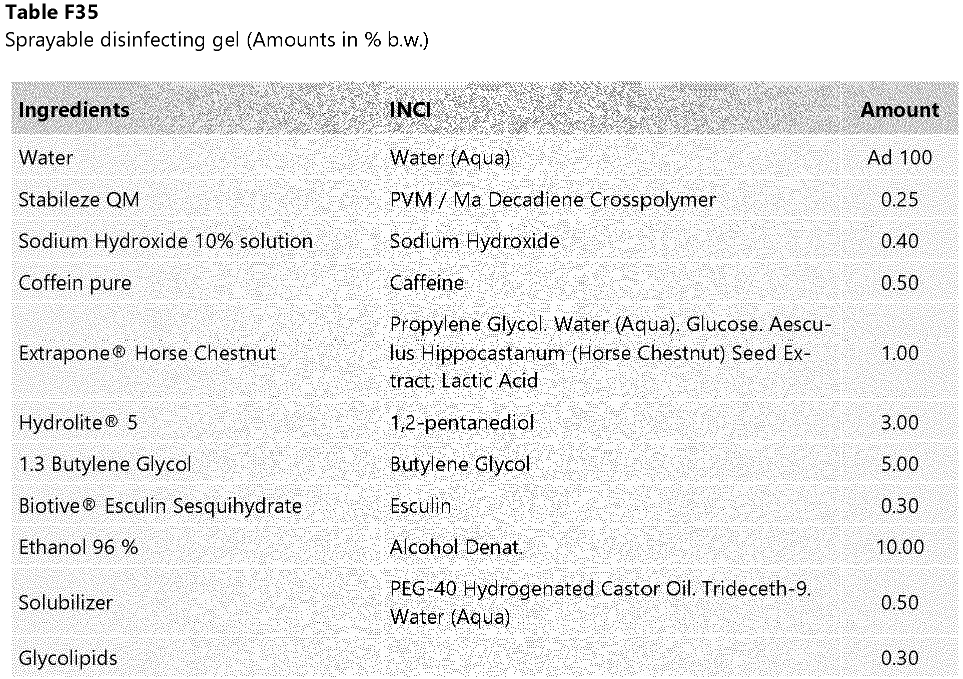

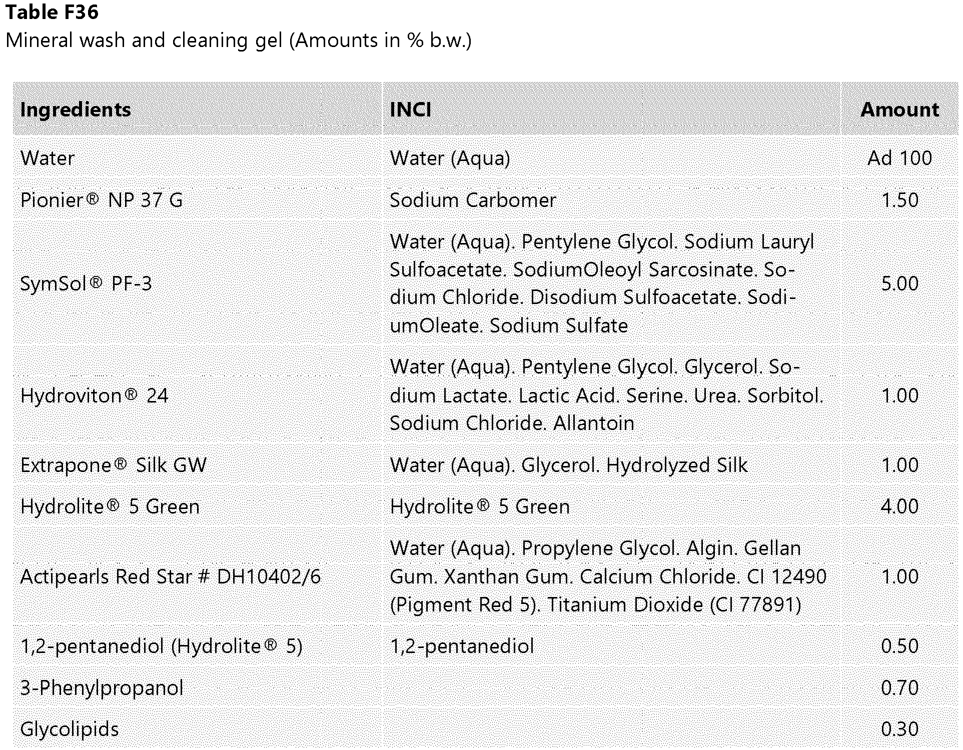

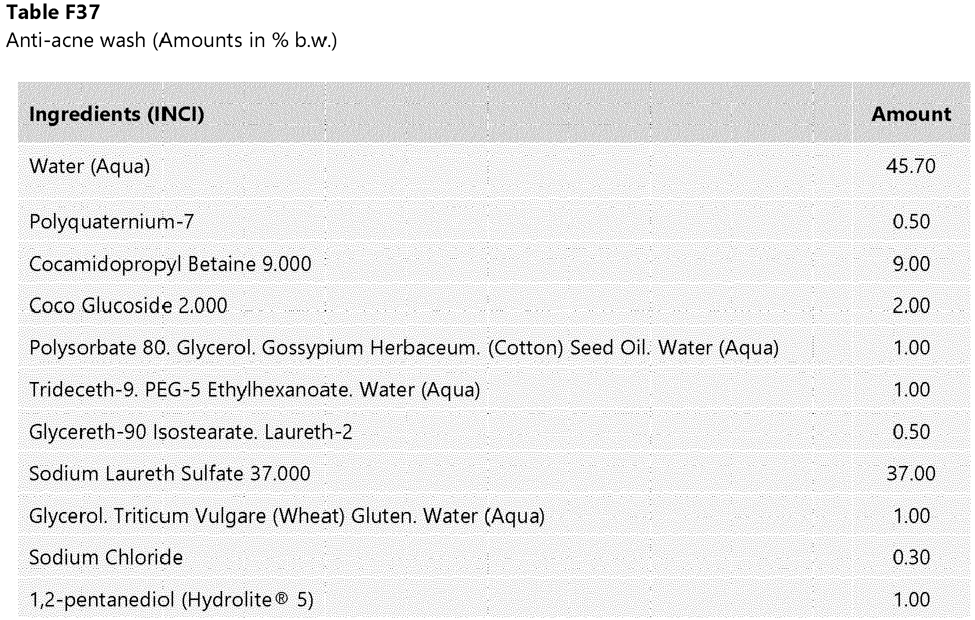

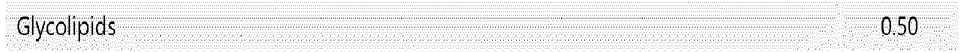

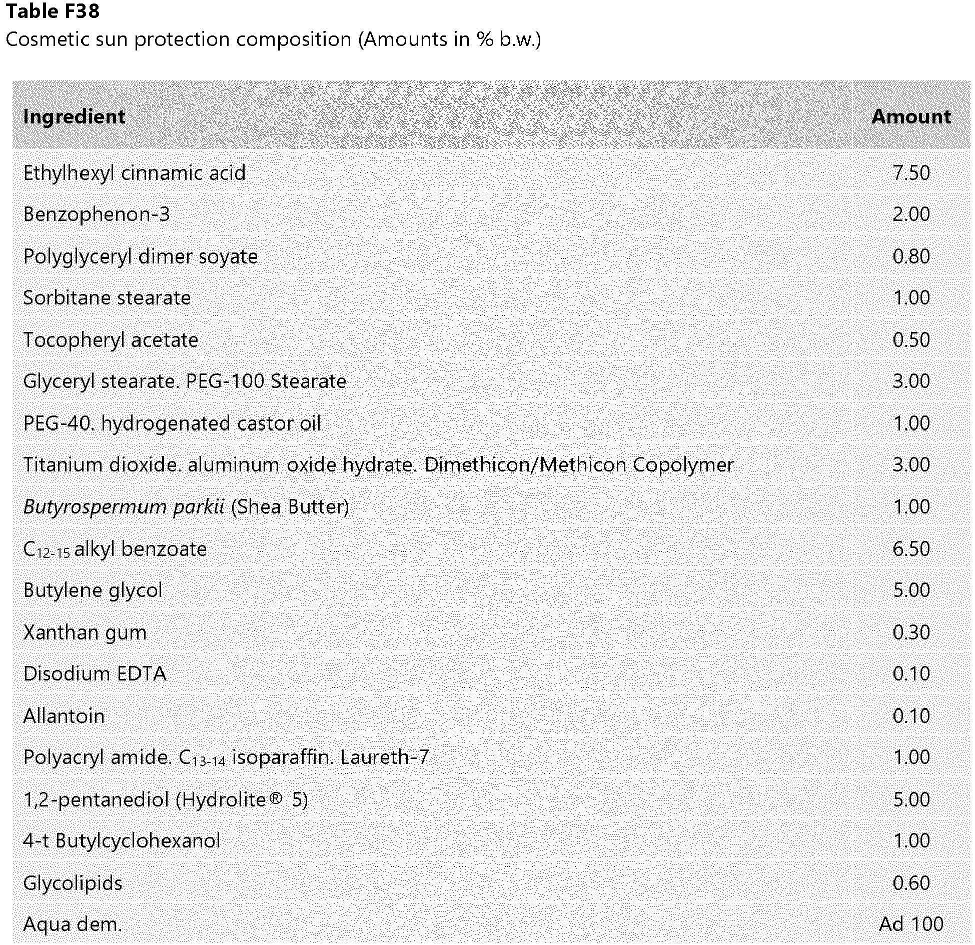

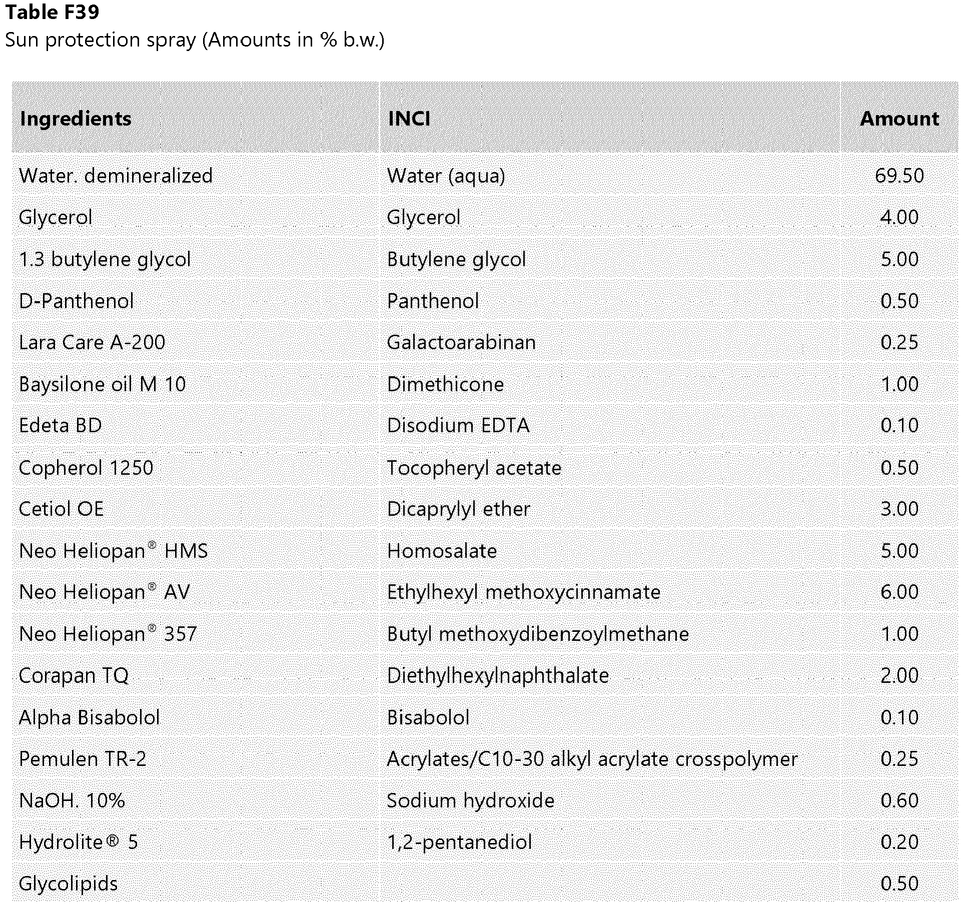

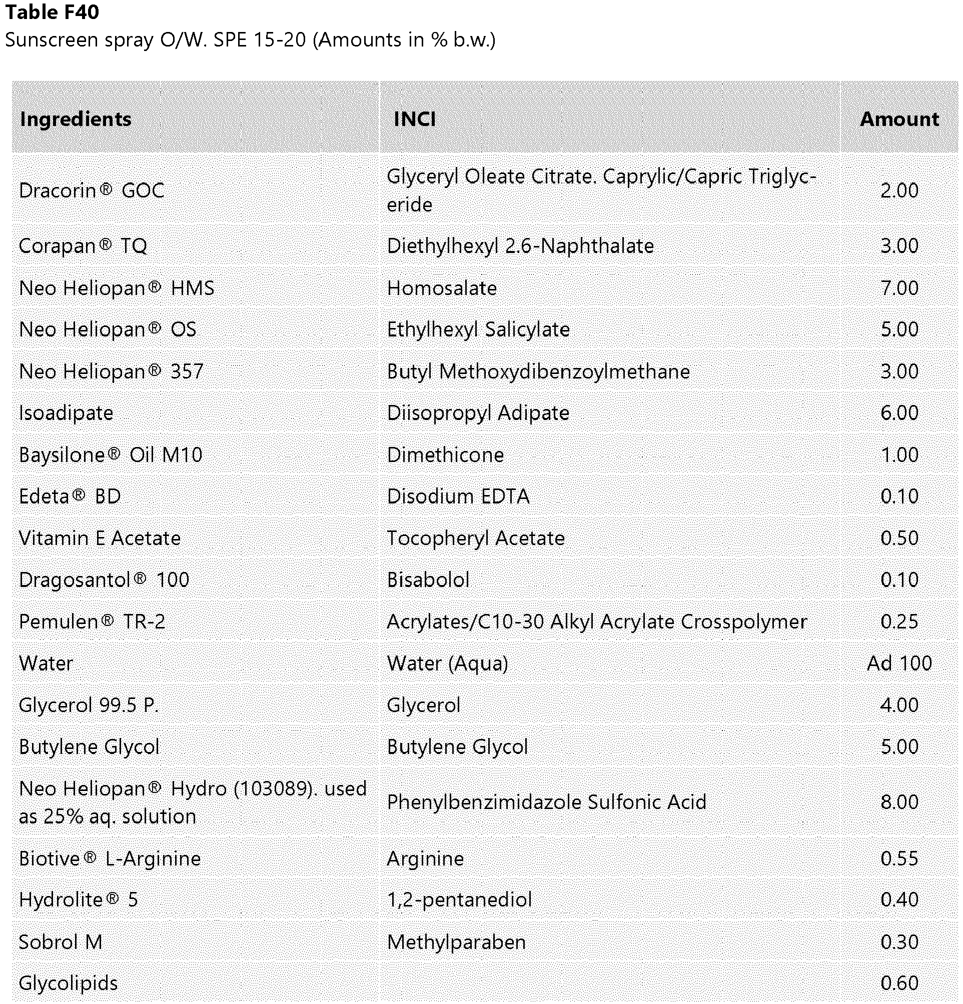

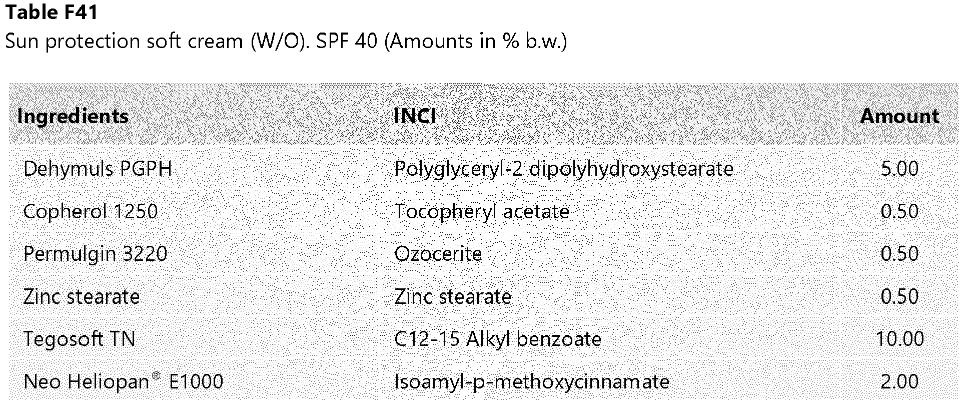

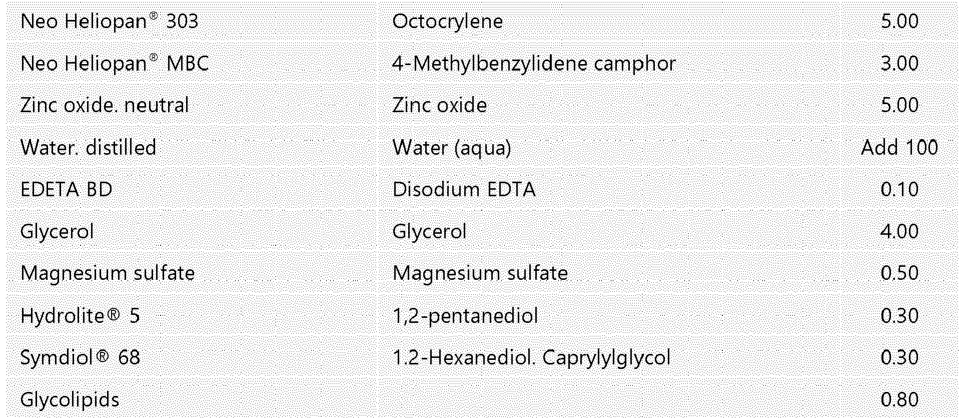

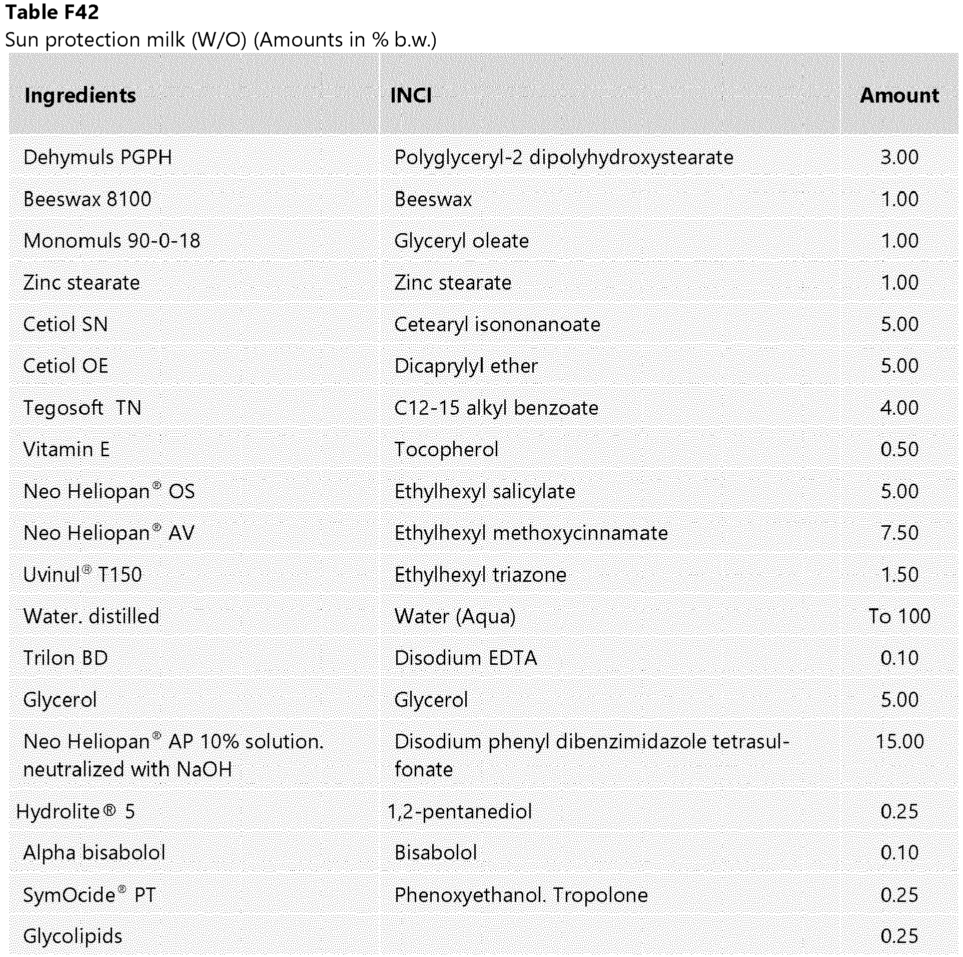

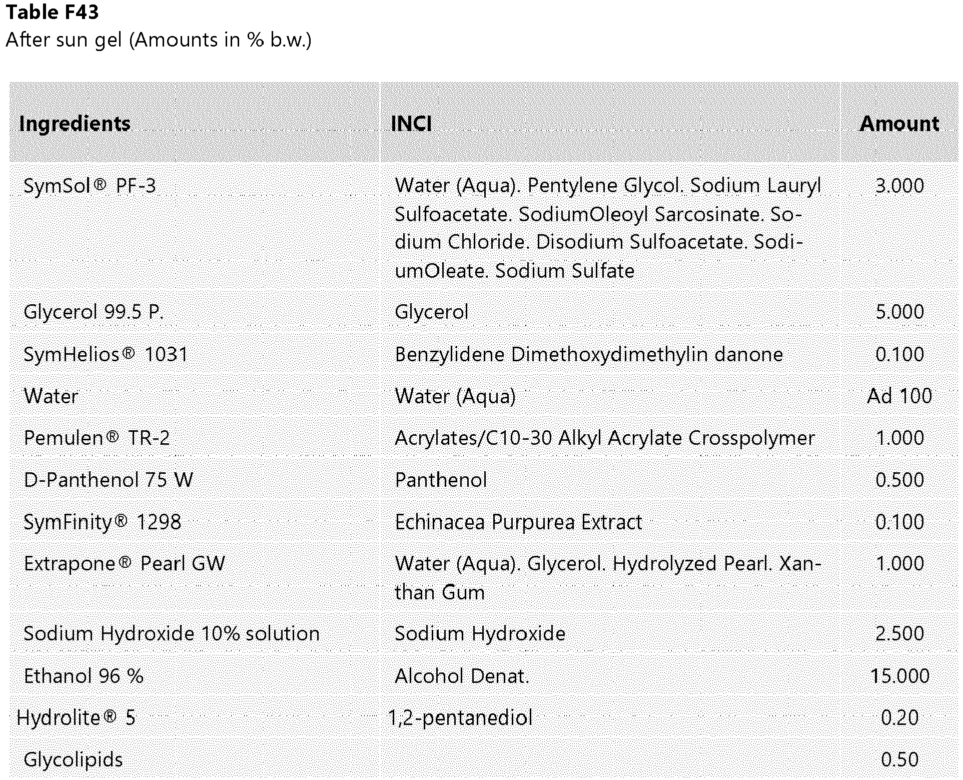

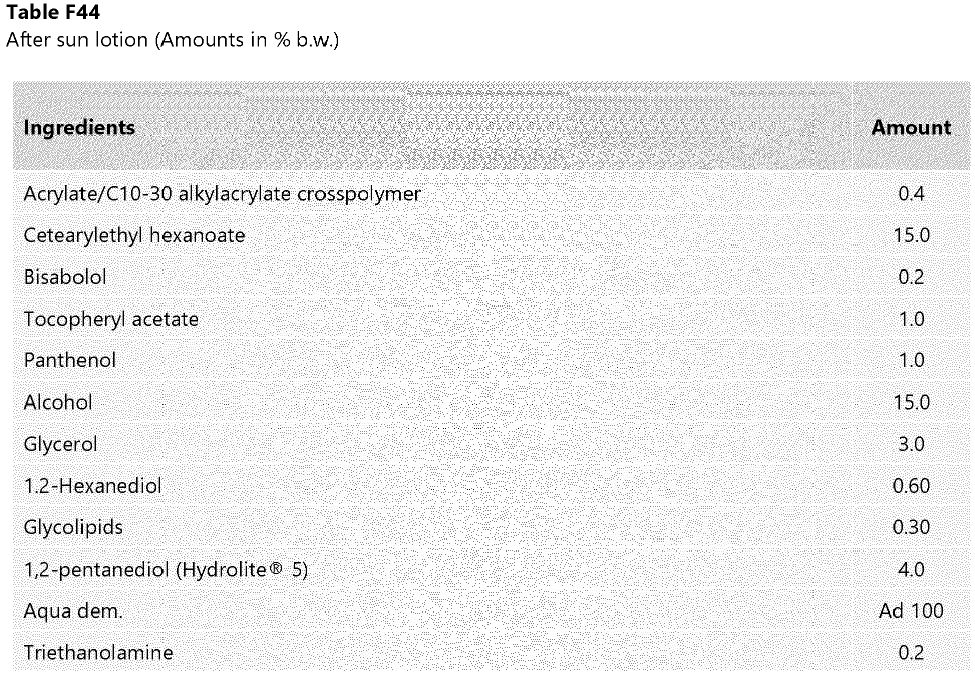

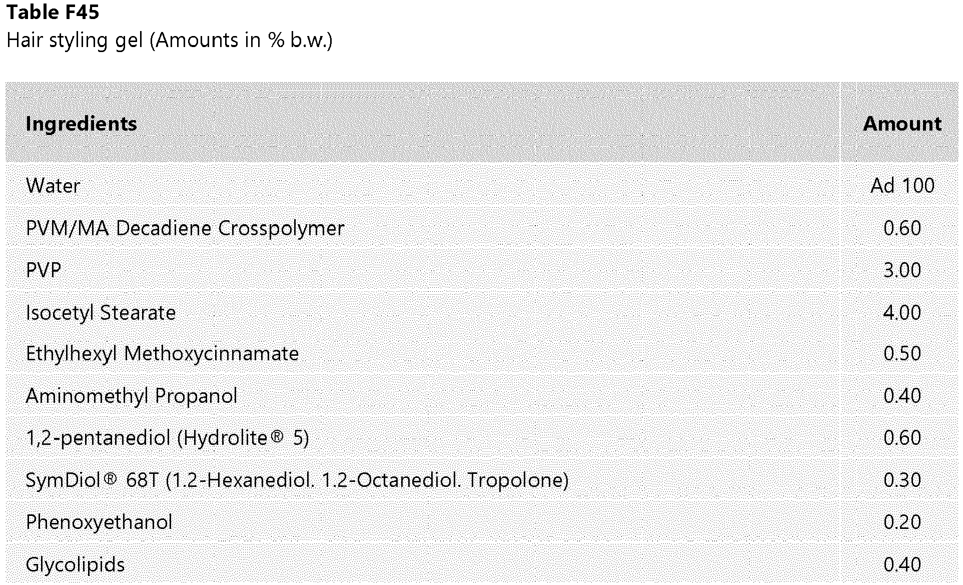

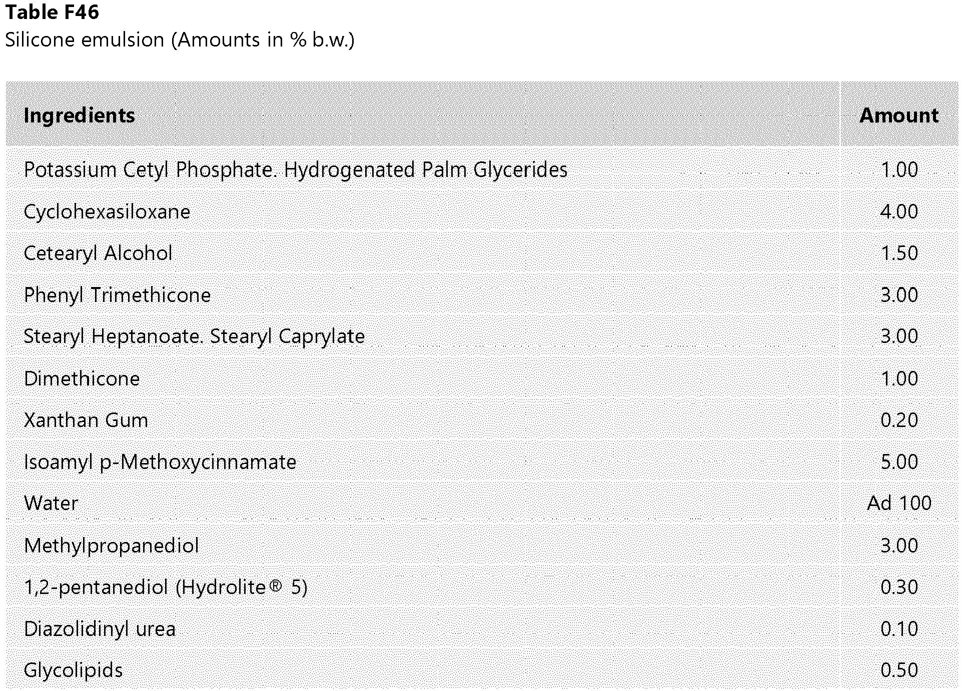

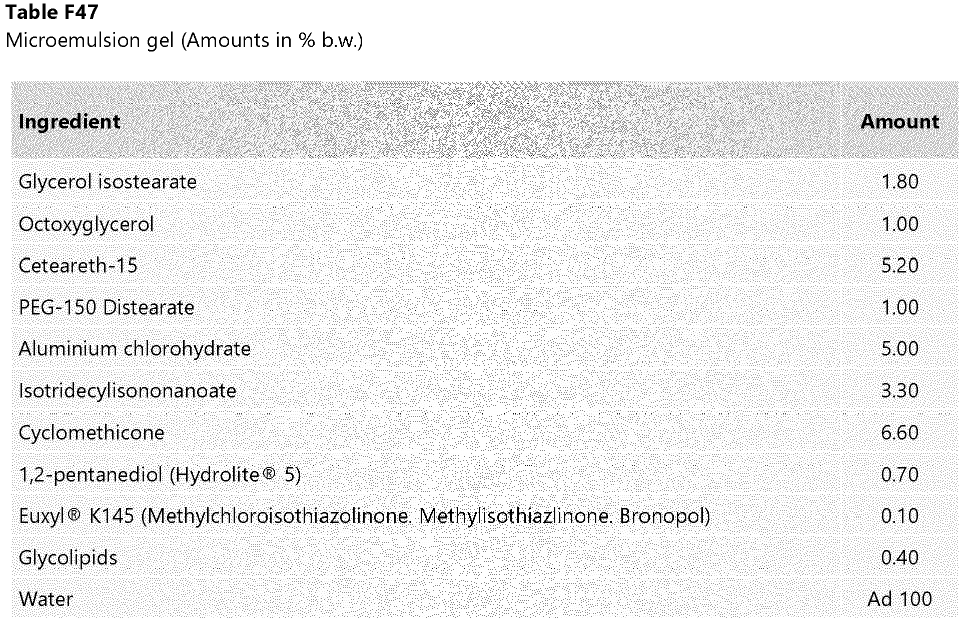

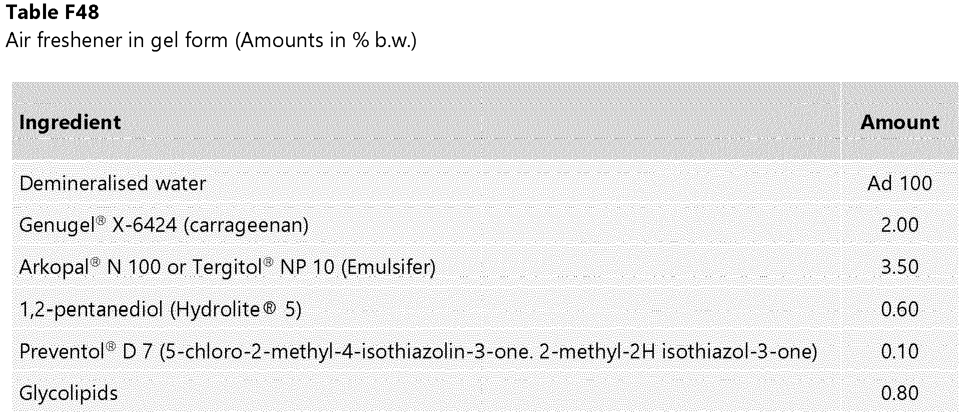

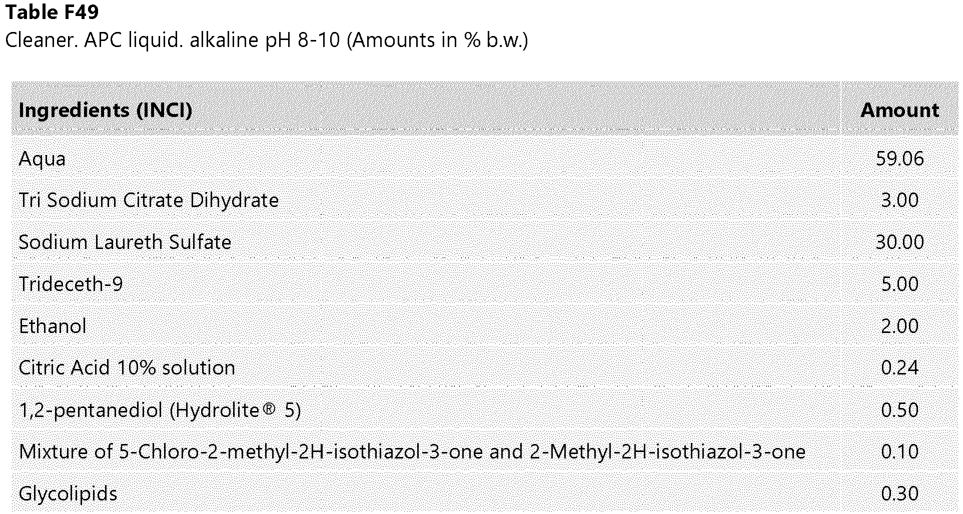

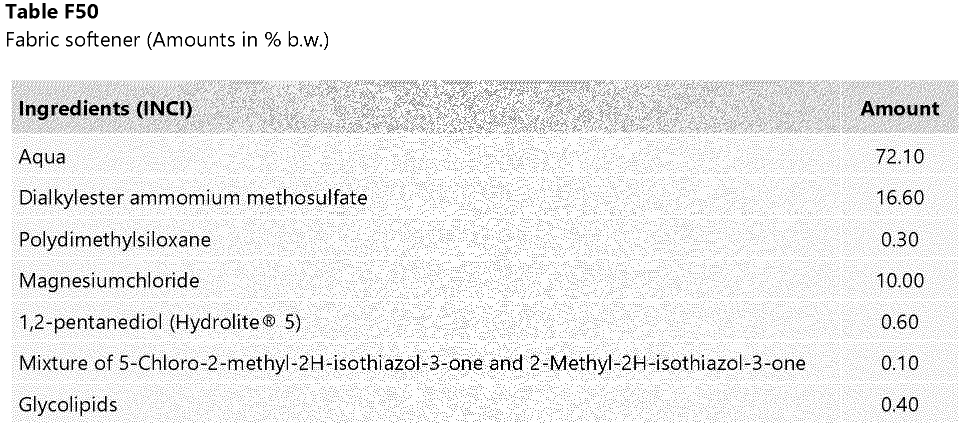

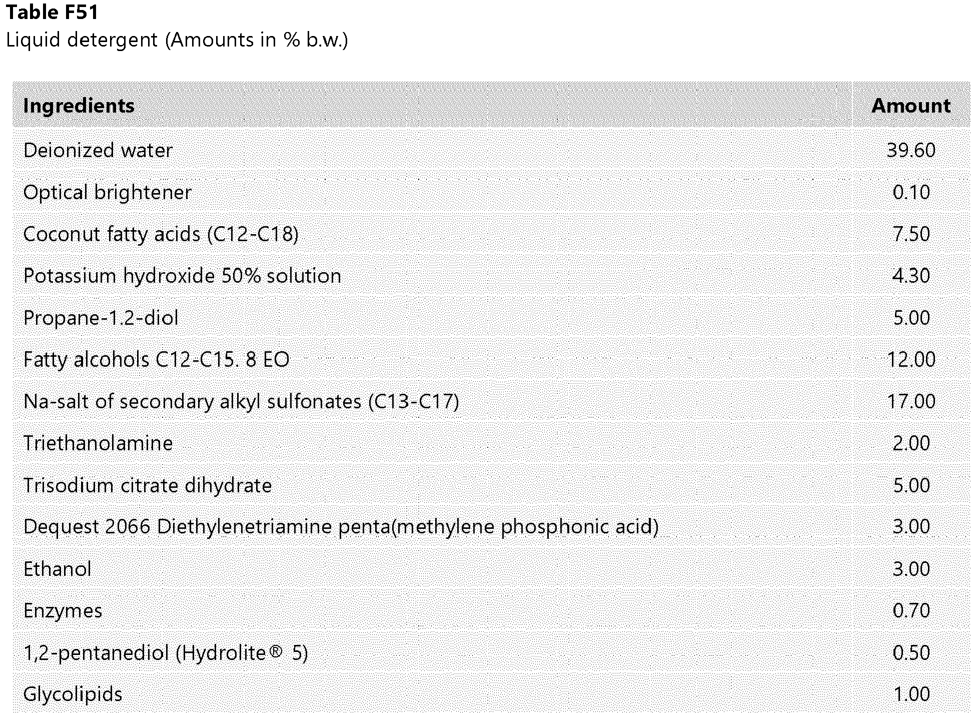

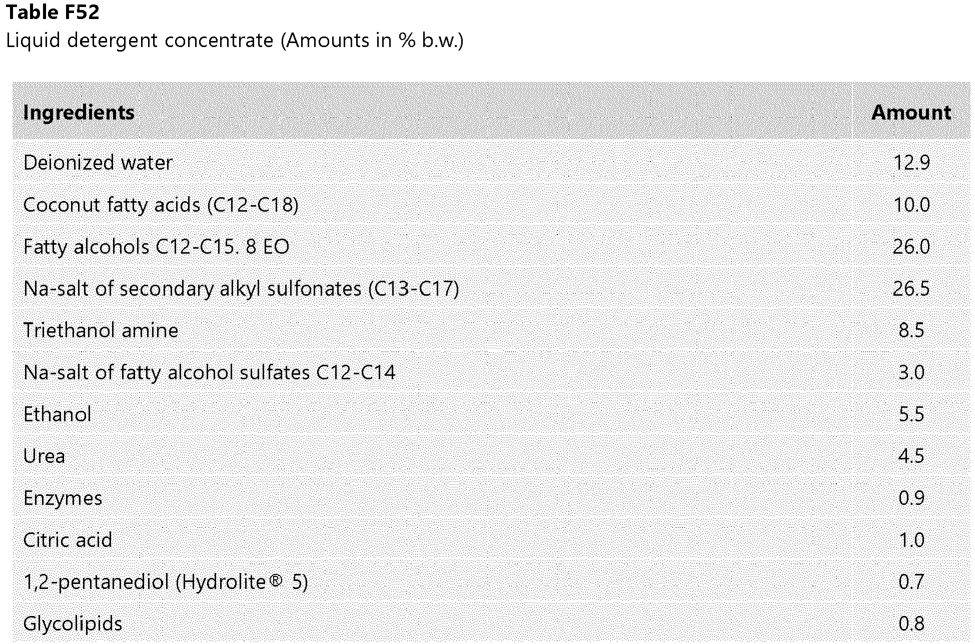

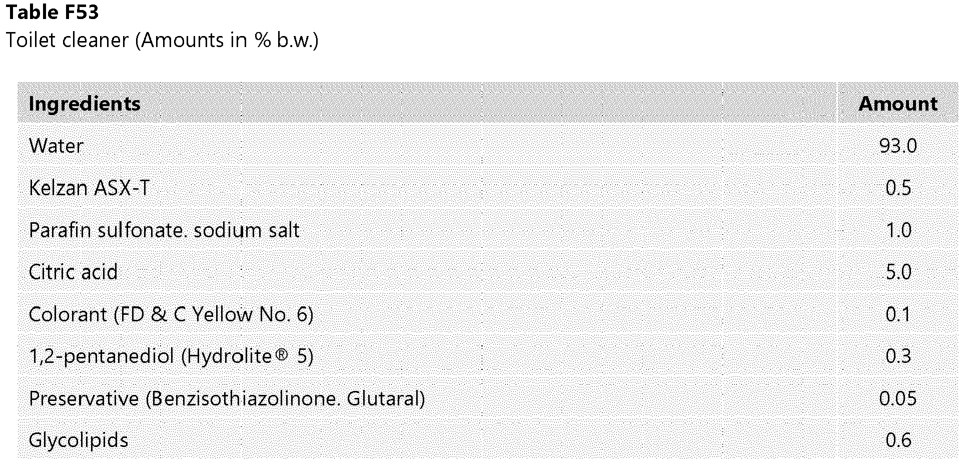

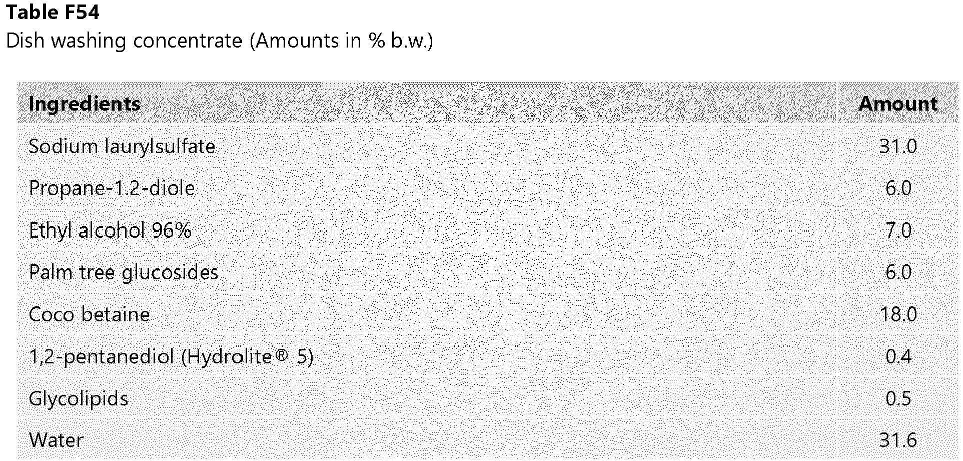

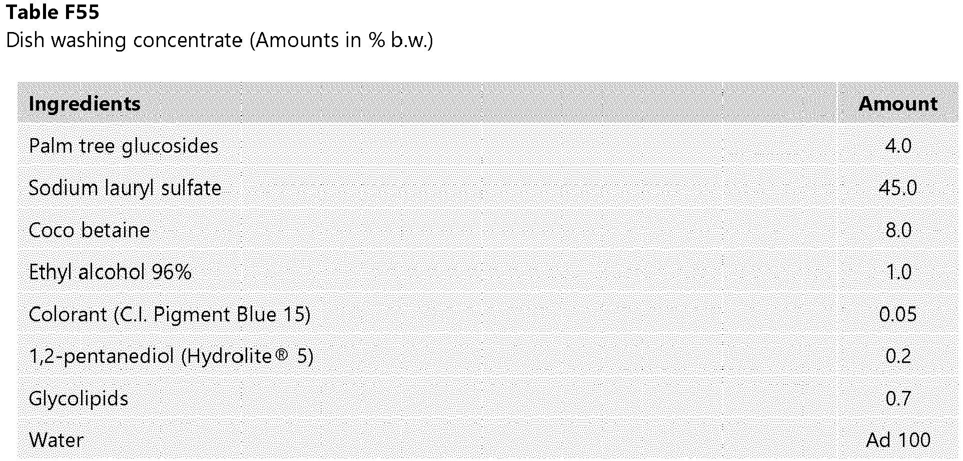

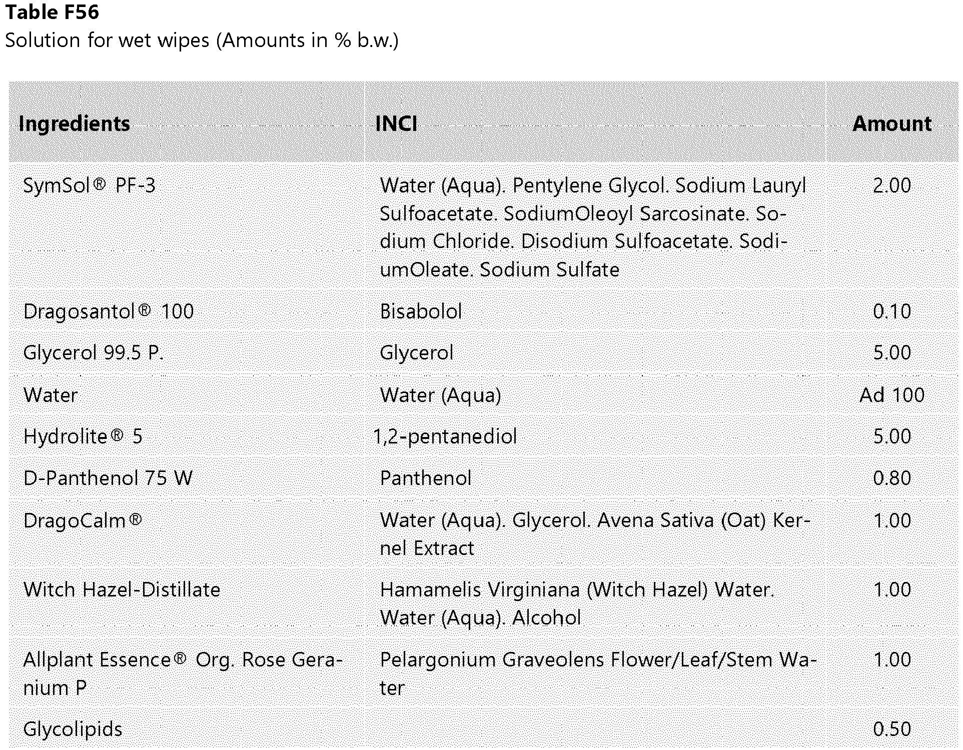

## Claims

1. A method for improving antimicrobial stability of a composition, against at least one of the following micro-organisms
• *Alicyclobacillus acidoterrestris*
• *Asaia siamensis*
• *Aspergillus brasiliensis*
• *Bacillus licheniformis*
• *Bacillus subtilis*
• *Brettanomyces sp*
• *Byssochlamys fulva*
• *Byssochlamys nivea*
• *Candida albicans*
• *Candida parapsilosis*
• *Clostridium perfringens*
• *Clostridium sporogenes*
• *Corynebacterium xerosis*
• *Dekkera naardenensis*
• *Enterococcus faecalis*
• *Gluconacetobacter liquefaciens*
• *Lactobacillus paracasei ssp. paracasei*
• *Lactobacillus plantarum ssp. plantarum*
• *Leuconostoc lactis*
• *Leuconostoc mesenteroides*
• *Malassezia furfur*
• *Neosartorya fischeri*
• *Penicillium expansum*
• *Penicillium paneum*
• *Saccharomyces cerevisiae*
• *Yarrowia lipolytica*
• *Zygosaccharomyces bailii*
• *Zygosaccharomyces rouxii*
comprising or consisting of the following steps:
(a) providing a composition in need of such improved antimicrobial stability;
(b) providing at least one glycolipid and optionally at least one other antimicrobial agent;
(c) adding a working amount of said at least one glycolipid and optionally said at least one other anti-microbial agent to said composition;
(d) optionally providing at least one other compound to said composition affecting solubility of compounds added in step (b) and (c);
wherein said glycolipid represents an ustilagic acid esterified with a linear C₁-C₄ aliphatic alcohol.

2. The method of Claim 1, wherein said composition is a cosmetic, detergent or food preparation.

3. The method of Claim 1, wherein said glycolipids follow formula (I) 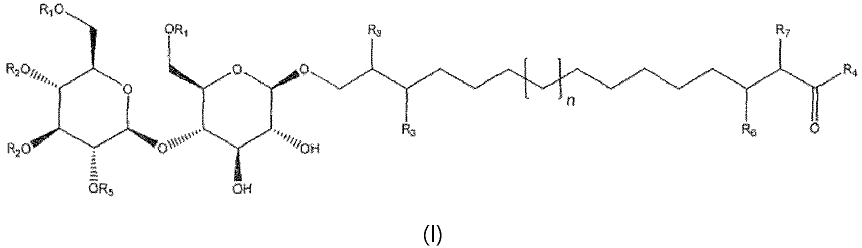 wherein independently from each other
n stands for 1 or 2,
R₁ represents hydrogen or -COCH₃;
R₂ represents H or 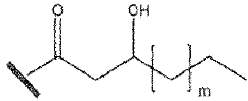
m = 1, 2 or 3;
R₃ represents hydrogen or -OH;
R₄ represents -OH or-OCH3;
R₅ represents hydrogen or 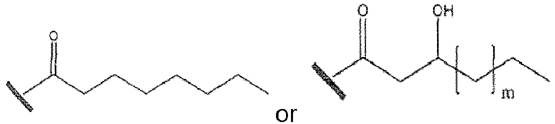
m = 1,2 or 3;
R₆ represents hydrogen or -OH;
R₇ represents hydrogen or -OH or =O;
or a salt of said glycolipid.

4. The method of Claim 1, wherein said glycolipids follow formula (II) 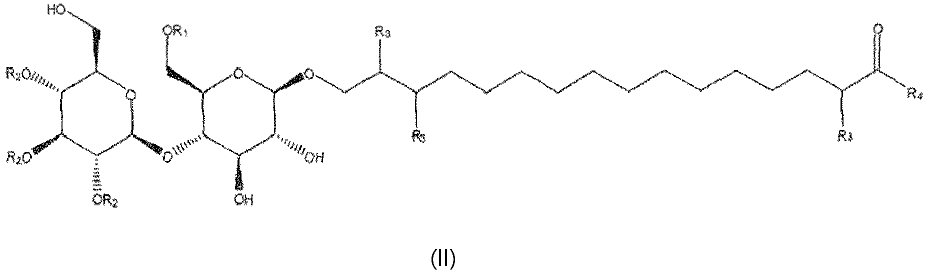 wherein independently from each other
R₁ represents hydrogen or -COCH₃;
R₂ represents H or 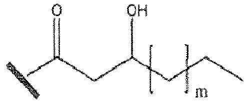
m = 1, 2 or 3;
R₃ represents hydrogen or -OH;
R₄ represents -OH or-OCH3;
or a salt of said glycolipid.

5. The method of Claim 1, wherein said glycolipids follow formula (III) 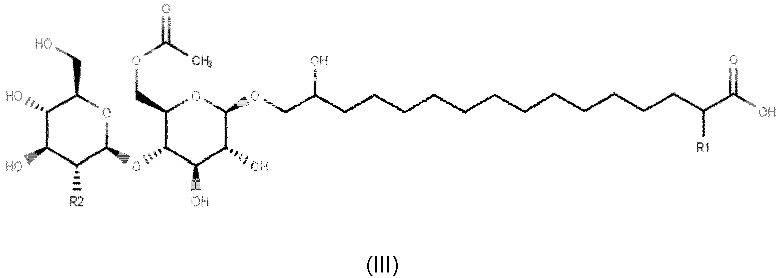 wherein independently from each other
R1 represents hydrogen or -OH;
R2 represents hydrogen or 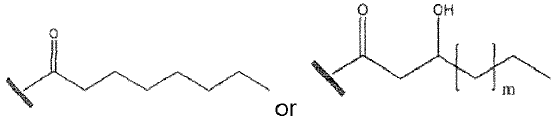
m = 1,2 or 3; or a salt of said glycolipid.

6. The method of Claim 5, wherein said glycolipids follow at least one the following formulae (1) to (6)
| **Com pou nd** | **Structure** | **Formula** | **Molecular weight** | **CAS** |
|---|---|---|---|---|
| 1 | 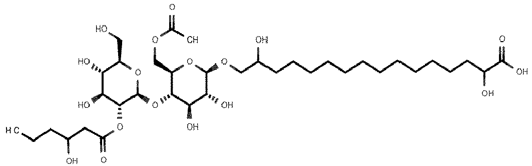 | C₃₆H₆₄ O₁₈ | 784,89 | 867578-35-6 |
| 2 | 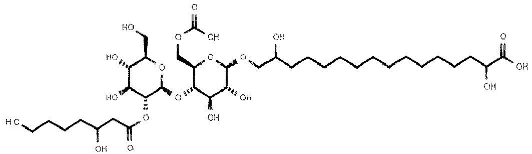 | C₃₈H₆₈ O₁₈ | 812,94 | 1427087-16-8 |
| 3 | 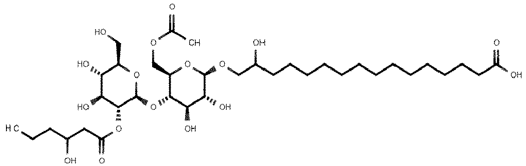 | C₃₆H₆₄ O₁₇ | 768,89 | 1427028-86-1 |
| 4 | 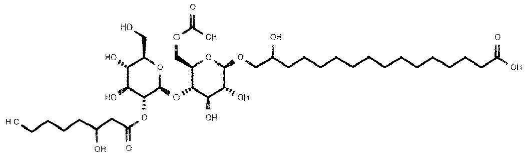 | C₃₈H₆₈ O₁₇ | 796,95 | |
| 5 | 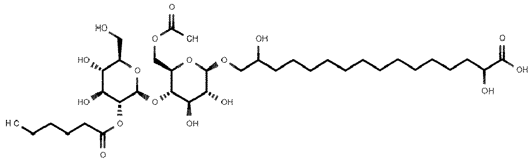 | C₃₆H₆₄ O₁₇ | 768,89 | |
| 6 | 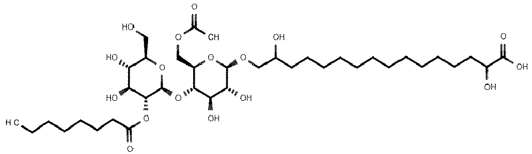 | C₃₈H₆₈ O₁₇ | 796,95 | 1427039-33-5 |
or a salt of said glycolipid.

7. The method of any of Claims 3 to 6 wherein said salts represent alkali salts, alkaline earth salts, ammonium salts, alkanol ammonium salts, glucammonium salts or mixtures thereof.

8. The method of Claim 1, wherein said at least one glycolipid is added in an amount of from about 1 to about 1,000 ppm - calculated on the composition.

9. The method of Claim 1, wherein said at least one glycolipid is added an amount of from about 10 to about 200 ppm - calculated on the composition.

10. The method of Claim 1, wherein secondary anti-microbial agents are added selected from the group consisting of benzoic acid and para-hydroxybenzoic acid, their esters and salts, benzyl benzoate, propionic acid and its salts, salicylic acid and its salts, 2,4-hexadienoic acid (sorbic acid) and its salts, levulinic acid and its salts, anisic acid and its salts, perillic acid and its salts, cinnamic acid and its salts, formaldehyde and paraformaldehyde, 4-hydroxy benzaldehyde, ortho-, meta-, and para-anisic aldehyde, cinnamic aldehyde, cinnamic alcohol, 2-hydroxybiphenyl ether and its salts, 2-zinc-sulfidopyridine N-oxide, inorganic sulfites and bisulfites, sodium iodate, chlorobutanolum, 4-ethylmercury-(II)5-amino-1,3-bis(2-hydroxybenzoic acid), its salts and esters, dehydracetic acid, formic acid, 1,6-bis(4-amidino-2-bromophenoxy)-n-hexane and its salts, the sodium salt of ethylmercury-(II)-thiosalicylic acid, phenylmercury and its salts, 10-undecylenic acid and its salts, 5-amino-1,3-bis(2-ethylhexyl)-5-methyl-hexahydropyrimidine, 5-bromo-5-nitro-1,3-dioxane, 2-bromo-2-nitro-1,3-propanediol, 2,4-dichlorobenzyl alcohol, N-(4-chlorophenyl)-N'-(3,4-dichlorophenyl)-urea, 4-chloro-m-cresol, 2,4,4'-trichloro-2'-hydroxy-diphenyl ether, 4-chloro-3,5-dimethylphenol, 1,1'-methylene-bis(3-(1-hydroxymethyl-2,4-dioximidazolidin-5-yl)urea), poly-(hexamethylenediguanide) hydrochloride, (Benzyloxymethoxy)-methanol hexamethylenetetramine, 1-(3-chloroallyl)-3,5,7-triaza-1-azonia-adamantane chloride, 1-(4-chlorophenoxy)-1-(1H-imidazol-1-yl)-3,3-dimethyl-2-butanone, 1,3-bis-(hydroxymethyl)-5,5-dimethyl-2,4-imidazolidinedione, 1,2-dibromo-2,4-dicyanobutane, 2,2'-methylene-bis(6-bromo-4-chlorophenol), bromochlorophene, mixture of 5-chloro-2-methyl-3(2H)-isothiazolinone, 2-methyl-3(2H)-isothiazolinone and with magnesium chloride and magnesium nitrate, 2-Octyl-2H-isothiazol-3-one, 1,2-benzisothiazol-3(2H)-one, 2-benzyl-4-chlorophenol, 3-(4-Chlorphenoxy)-1,2-propanediol (Chlorphenesin), 2-chloroacetamide, chlorhexidine, chlorhexidine acetate, chlorhexidine gluconate, chlorhexidine hydrochloride, N-alkyl(C₁₂-C₂₂)trimethyl-ammonium bromide and chloride, 4,4-dimethyl-1,3-oxazolidine, N-hydroxymethyl-N-(1,3-di(hydroxymethyl)-2,5-dioxoimidazolidin-4-yl)-N'-hydroxy-methylurea, 1,6-bis(4-amidino-phenoxy)-n-hexane and its salts, glutaraldehyde, 5-ethyl-1-aza-3,7-dioxabicyclo(3.3.0)octane, 3-(4-chlorophenoxy)-1,2-propanediol, hyamines, alkyl-(C₈-C₁₈)-dimethyl-benzyl-ammonium chloride, alkyl-(C₈-C₁₈)-dimethyl-benzylammonium bromide, alkyl-(C₈-C₁₈)-dimethylbenzyl-ammonium saccharinate, benzyl hemiformal, 3-iodo-2-propynyl butylcarbamate, sodium hydroxymethyl-aminoacetate or sodium hydroxymethyl-aminoacetate, imidazolidinylurea, diazolidinylurea, sodium hydroxymethylglycinate, DMDM hydantoin, Tropolone, (Ethylendioxy)dimethanol, 2-Brom-2-(brommethyl)pentandinitril, N-(3-Aminopropyl)-N-dodecylpropan-1,3-diamin, α,α',α"-trimethyl-1,3,5-triazine-1,3,5(2H,4H,6H)-triethanol, pyridine-2-thiol-1-oxide, sodium salt, Tetrahydro-1,3,4,6-tetrakis(hydroxymethyl)imidazo[4,5-d]imidazol-2,5(1H,3H)-dion, 1,3-bis(hydroxymethyl) -1-(1,3,4-tris(hydroxy-methyl)-2,5-dioxoimidazolidin-4-yl)urea (Diazolidinyl Urea), 1,3-Bis(hydroxy-methyl)-5,5-dimethylimidazolidine-2,4-dione, 3-Acetyl-2-hydroxy-6-methyl-4H-pyran-4-one, cetyl pyridium chloride, caprylhydroxamic acid, sorbohydroxamic acid and their mixtures; 1,3-propanediol, methyl propanediol, 1,2-pentanediol, 1,2-hexanediol, 1,2-octanediol, 1,2-decanediol, 1,5-pentanediol, 1,6-hexanediol, 1,8-octanediol, 1,2-decanediol, ethylhexylglycerin, hexoxy-propan-1,2-diol, heptoxy- propan-1,2-diol, octoxy-propan-1,2-diol, 3-phenoxy-propan-1,2-diol, 3-benzyloxy-propan-1,2-diol, 3-phenylethyloxy-propan-1,2-diol, 3-phenylpropyloxy-propan-1,2-diol, 3-methylbenzyloxy-propan-1,2-diol, sorbitan caprylate, triclosan, climbazole, Octopirox (1-hydroxy-4-methyl-6-(2,4,4-trimethylpentyl)-2(1H)-pyridone, 2-aminoethanol), chitosan, farnesol, 2-butyloctanoic acid, 2-Benzylheptan-1-ol, glycerol monolaurate, bis(2-pyridylthio)zinc 1,1'-dioxide, N,N'-(decane-1,10-diyldipyridin-1-yl-4-ylidene)-dioctan-1-amine dihydrochloride (octenidine dihydrochloride), thymol, eugenol, benzyl alcohol, 2-phenyethyl alcohol, 3-phenyl propanol, 2-phenoxyethanol, 1-phenoxy-propan-2-ol, 3-phenoxypropanol, benzyloxymethanol, 4-hydroxyacetophenone and mixtures thereof.

11. The method of Claim 1, wherein solubility affecting agents are selected from the group consisting of
∘ saponines obtainable from the plant genera
• *Quillaja,*
• *Yucca,*
• *Dioscorea,*
• *Paris,*
• *Hosta,*
• *Agave,*
• *Asparagus,*
• *Allium,*
• *Sapindus,*
• *Aesculus,*
• *Hedera,*
• *Blighia,*
• *Dipsacus,*
• *Aralia* and/or
• *Anemone*
or mixtures thereof,
o emulsifiers E400 to E499 or E999 approved for food preparations in the EU, or mixtures thereof
o synthetic food grade emulsifiers selected from the group consisting of lecithin; polyglycerol esters (PGE), polysorbates, stearoyl lactylates, propylene glycol esters (PGMS), sucrose esters; polyglycerol Polyricinoleate (PGPR); ammonium phosphatide (AMP); mono and diglycerides of C₆-C₂₂ saturated or unsaturated fatty acids and mixtures thereof.

12. The method of Claim 1, wherein the pH value of the composition is adjusted by inorganic or organic acids to about 1 to about 4.

13. The use of glycolipids as antimicrobial agents against at least one of the following micro-organisms
• *Alicyclobacillus acidoterrestris*
• *Asaia siamensis*
• *Aspergillus brasiliensis*
• *Bacillus licheniformis*
• *Bacillus subtilis*
• *Brettanomyces sp*
• *Byssochlamys fulva*
• *Byssochlamys nivea*
• *Candida albicans*
• *Candida parapsilosis*
• *Clostridium perfringens*
• *Clostridium sporogenes*
• *Corynebacterium xerosis*
• *Dekkera naardenensis*
• *Enterococcus faecalis*
• *Gluconacetobacter liquefaciens*
• *Lactobacillus paracasei ssp. paracasei*
• *Lactobacillus plantarum ssp. plantarum*
• *Leuconostoc lactis*
• *Leuconostoc mesenteroides*
• *Malassezia furfur*
• *Neosartorya fischeri*
• *Penicillium expansum*
• *Penicillium paneum*
• *Saccharomyces cerevisiae*
• *Yarrowia lipolytica*
• *Zygosaccharomyces bailii*
• *Zygosaccharomyces rouxii*
for preserving compositions at a pH of about 1 to about 4, wherein said glycolipid represents an ustilagic acid or an ester of ustilagic acid esterified with a linear C₁-C₄ aliphatic alcohol.

14. The use according to Claim 13, wherein said at least one ustilagic acid or its ester is added in a working amount of from about 1 to about 1,000 ppm - calculated on the composition.

15. The use according to Claim 13, wherein said composition is a cosmetic, detergent or food preparation.
